(19) **Europäisches Patentamt / European Patent Office / Office européen des brevets**

(11) **EP 1 914 308 A1**

(12) **EUROPEAN PATENT APPLICATION**
published in accordance with Art. 153(4) EPC

(43) Date of publication:
**23.04.2008 Bulletin 2008/17**

(21) Application number: **06767462.2**

(22) Date of filing: **21.06.2006**

(51) Int Cl.:
**C12N 15/09** *(2006.01)*     **A01H 1/00** *(2006.01)*
**A01H 5/00** *(2006.01)*

(86) International application number:
**PCT/JP2006/312845**

(87) International publication number:
**WO 2006/137574 (28.12.2006 Gazette 2006/52)**

(84) Designated Contracting States:
**IT**

(30) Priority: **22.06.2005 JP 2005182251**

(71) Applicant: **Incorporated Administrative Agency National**
**Agriculture and Food Research Organisation Ibaraki**
**305-8517 (JP)**

(72) Inventors:
• **YOSHIDA, Midori,**
**Nat. Agricultural Research Center**
**Sapporo-shi,**
**Hokkaid 0628555 (JP)**

• **KAWAKAMI, Akira,**
**Nat. Agricultural Research Center**
**Tsukuba-shi,**
**Ibaraki 3058666 (JP)**

• **SATO, Yutaka,**
**Nat. Agricultural Research Center**
**Sapporo-shi,**
**Hokkaid 0628555 (JP)**

(74) Representative: **Woods, Geoffrey Corlett**
**J.A. KEMP & CO.**
**Gray's Inn**
**14 South Square**
**London WC1R 5JJ (GB)**

(54) **COLD-HARDY PLANT AND METHOD FOR DEVELOPMENT OF THE SAME**

(57) The present invention relates to a transgenic rice plant having enhanced chilling tolerance which contains a fructosyltransferase gene, a method for enhancing the chilling tolerance of a rice plant via introduction of the gene into a rice cell, and use of the transgenic rice plant for alleviating chilling damage in rice cultivation.

**EP 1 914 308 A1**

**Description**

Technical Field

The present invention relates to plants with enhanced chilling tolerance and a method for enhancing chilling tolerance of plants.

Background Art

Crop cultivation in cold regions is problematic in that damages associated with low temperatures tend to occur, such as frost damages during early spring or late autumn, cool weather damages due to cool temperatures during summer, and freezing damages or snow damages during winter. Hence, various crop plants with enhanced low temperature resistance have been aggressively developed conventionally.

Low temperature tolerance is broadly classified into chilling tolerance and cold tolerance in view of temperature factor. Chilling tolerance is tolerance to low temperatures within the positive temperature range (i.e., cool temperatures) to which crop plants are exposed from early spring to autumn. In general, crop plants tend to experience damages such as growth delay, poor ripening, and sterility due to cool temperatures during the seasons. Therefore, high-level chilling tolerance of crop plants is of advantage for reducing decreases in yield due to such damages. Meanwhile, cold tolerance is tolerance to freezing temperatures (0°C or less) to which, in general, overwintering crop plants are exposed during winter. The cold tolerance encompasses freezing tolerance (ability to avoid freezing damages at 0°C or less), snow tolerance (ability to avoid freezing damages at 0°C or less and achieve long-term survival even under dark), and the like. Crop plants exerting cold tolerance can survive even under freezing temperature conditions while minimizing damages due to freezing.

Fructans are known as substances that contribute to plant cold tolerance. Fructans are energy sources during overwintering and are polysaccharides having antifreezing and drought tolerance functions. Studies have been conducted to improve the freezing tolerance and drought tolerance of plants via introduction of bacterial or plant fructosyltransferase genes into the plants. As a result, improvement of the freezing tolerance and drought tolerance by the introduction of the fructosyltransferase genes has been reported in tobacco plant (Konstantinova T. et al., Plant Sci. (2002) 163: pp. 157-164) and perennial ryegrass (Hisano H. et al., Plant Sci. (2004) 167: pp. 861-868). A fructosyltransferase gene that contributes to high-level cold tolerance of winter wheat has also been isolated (JP Patent Publication (Kokai) No. 2000-350583A and Kawakami A. and Yoshida M., Biosci. Biotechnol. Biochem. (2002) 66 (11), pp. 2297-2305).

During early spring cultivation and direct seeding cultivation of rice plants that are non-overwintering crop plants, rice plants often encounter low temperatures below 10°C during their early growth stage, so that rice plants frequently experience significant low-temperature damages leading to their death (Tajima et al., "Physiological Study on Inhibition of the Growth of Rice Due to Low Temperatures," Agricultural Research Report (Nogikenho), (1983) 34: pp. 69-111). Furthermore, when rice plants at the booting stage encounter low temperatures during summer, their pollen formation is inhibited so that they become male sterile and the resulting yield is significantly lowered (Satake T. and Hayase H., Proc. Crop Sci. Japan (1970) 39: pp. 468-473). Development of rice plants with enhanced chilling tolerance that is exerted during such growth stage has been actively attempted. However, for breeding methods using existing genetic resources as materials, expectations are limited regarding the significant extent of improvement in chilling tolerance.

Disclosure of the Invention

An object of the present invention is to provide plants having tolerance to stress of low temperatures within the positive temperature range, i.e., chilling tolerance, and a method for enhancing the chilling tolerance of plants.

The present inventors have conducted concentrated studies in order to attain the above objects. The present inventors have thus discovered that strong chilling tolerance (tolerance to low temperatures within the positive temperature range) is exerted by a transgenic rice plant into which a sucrose:sucrose 1-fructosyltransferase (1-SST) gene or a sucrose:fructan 6-fructosyltransferase (6-SFT) gene, each of which is a fructosyltransferase gene, has been introduced. The present inventors have thus completed the present invention based on the finding. "Fructan" is a generic term for, mainly, tri- or higher saccharides which are formed via polymerization of fructoses to sucrose. Polysaccharides formed via polymerization of fructose units alone are also included among the examples of fructan.

The present invention encompasses the following [1] to [3].

[1] A transgenic rice plant having enhanced chilling tolerance, comprising at least one of the following fructosyltransferase genes (a) to (f):

(a) a gene comprising the nucleotide sequence shown in SEQ ID NO: 1 or 3;

(b) a gene comprising DNA that hybridizes under stringent conditions to DNA comprising the nucleotide sequence shown in SEQ ID NO: 1 or 3 and encodes a protein having fructan synthetic activity;

(c) a gene encoding a protein that comprises the amino acid sequence shown in SEQ ID NO: 2 or 4;

(d) a gene encoding a protein that comprises an amino acid sequence derived from the amino acid sequence shown in SEQ ID NO: 2 or 4 by deletion, substitution, or addition of one or several amino acids and has fructan synthetic activity;

(e) a gene comprising a nucleotide sequence that exhibits 85% or more identity with the nucleotide sequence shown in SEQ ID NO: 1 or 3 and encoding a protein that has fructan synthetic activity; and

(f) a gene encoding a protein that comprises an amino acid sequence exhibiting 85% or more identity with the amino acid sequence shown in SEQ ID NO: 2 or 4 and has fructan synthetic activity.

The chilling tolerance enhanced in the transgenic rice plant is preferably seedling-stage chilling tolerance and/or booting-stage chilling tolerance.

[2] A method for enhancing chilling tolerance of a rice plant, comprising introducing at least one of the following fructosyltransferase genes (a) to (f) into a rice cell:

(a) a gene comprising the nucleotide sequence shown in SEQ ID NO: 1 or 3;

(b) a gene comprising DNA that hybridizes under stringent conditions to DNA comprising the nucleotide sequence shown in SEQ ID NO: 1 or 3 and encodes a protein having fructan synthetic activity;

(c) a gene encoding a protein that comprises the amino acid sequence shown in SEQ ID NO: 2 or 4;

(d) a gene encoding a protein that comprises an amino acid sequence derived from the amino acid sequence shown in SEQ ID NO: 2 or 4 by deletion, substitution, or addition of one or several amino acids and has fructan synthetic activity;

(e) a gene comprising a nucleotide sequence that exhibits 85% or more identity with the nucleotide sequence shown in SEQ ID NO: 1 or 3 and encoding a protein that has fructan synthetic activity; and

(f) a gene encoding a protein that comprises an amino acid sequence exhibiting 85% or more identity with the amino acid sequence shown in SEQ ID NO: 2 or 4 and has fructan synthetic activity.

Chilling tolerance that is enhanced by this method is preferably seedling-stage chilling tolerance and/or booting-stage chilling tolerance.

[3] Use of the transgenic rice plant according to [1] or [2] above for alleviating chilling damages in rice plant cultivation. In this use, in particular, chilling damages occurred during seedling stage and/or booting stage of rice plants can be effectively alleviated.

The transgenic rice plant of the present invention exerts high tolerance to low temperatures within the positive temperature range. Therefore, through the use of the transgenic rice plant of the present invention, cool weather damages such as death due to low temperatures during summer can be alleviated, and thereby enabling rice cultivation in which sufficient growth amounts and yields can be obtained even in unsuitable regions for rice cultivation where cool weather damages tend to occur. The use of the method for enhancing the chilling tolerance of the rice plant of the present invention can confer high-level chilling tolerance to existing rice cultivars.

The disclosure in the description and/or drawings of Japanese Patent Application No. 2005-182251, from which the present application claims priority, is incorporated herein.

Brief Description of the Drawings

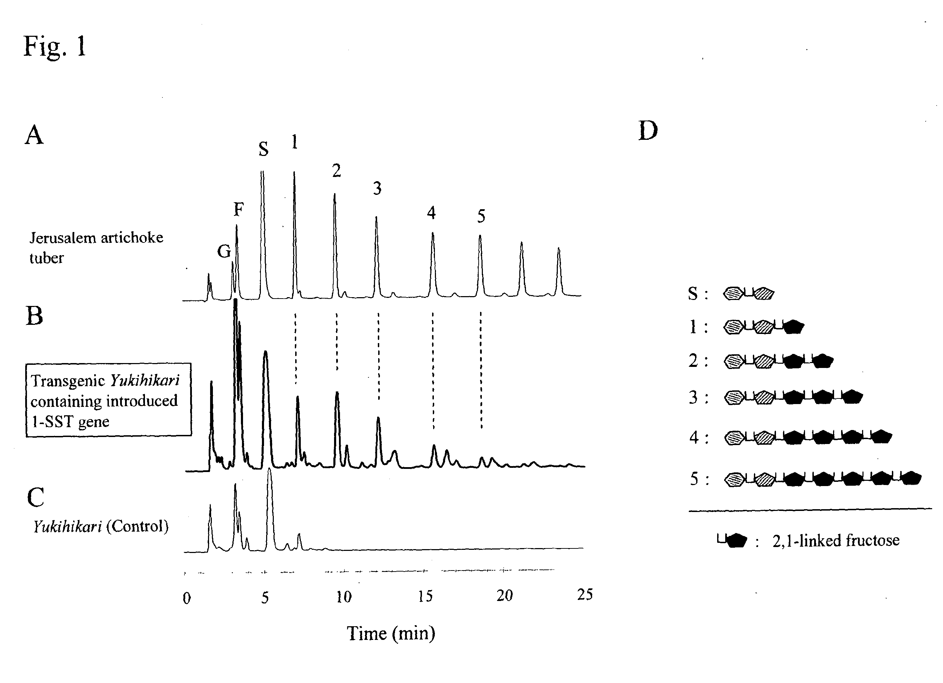

Fig. 1 shows the chromatogram of soluble sugars extracted from transgenic rice plants containing introduced 1-SST gene [(B)], the chromatogram of soluble sugars contained in each of extracts obtained from Jerusalem artichoke tubers [(A)] and original cultivar, *Yukihikari* [(C)] respectively, and structures of fructans that appeared as peaks in the chromatogram [(D)]. G, glucose; F, fructose; and S, sucrose. 1 to 5 indicate fructans with degrees of polymerization ranging from 3 to 7.

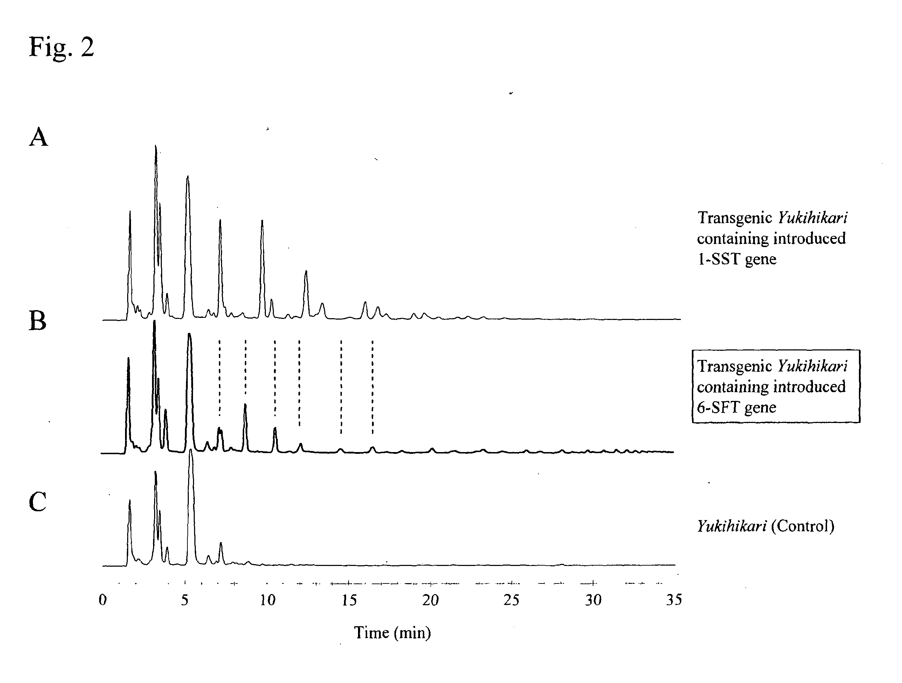

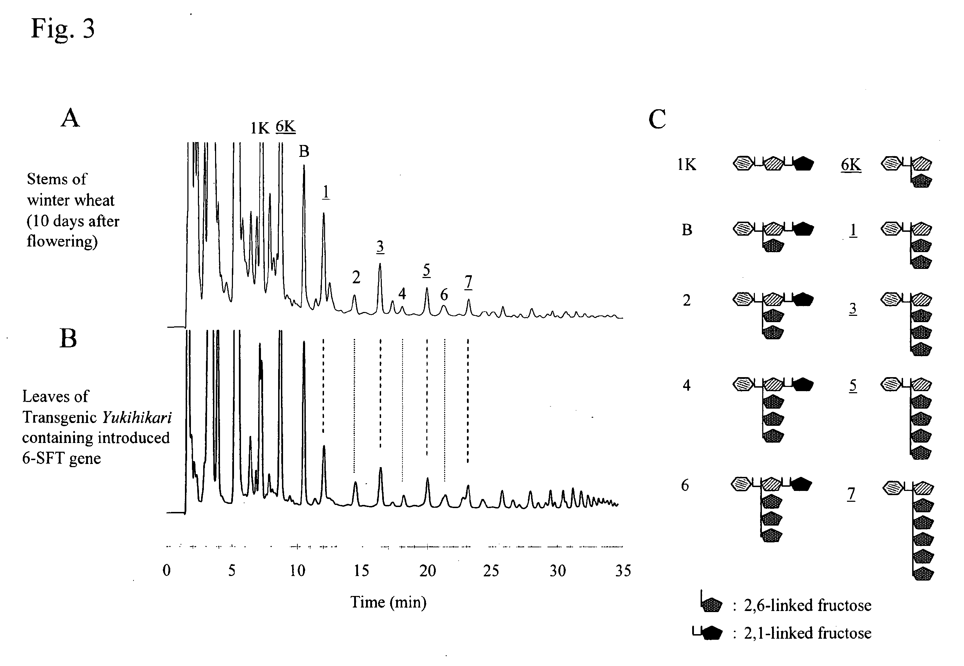

Fig. 2 shows a comparison of the chromatogram of soluble sugars extracted from transgenic rice plants containing introduced 1-SST gene with that of soluble sugars extracted from transgenic rice containing introduced 6-SFT gene. Fig. 3 shows the chromatogram of soluble sugars extracted from transgenic rice plants containing introduced 6-SFT gene, and structures of fructans contained in the extracts obtained from winter wheat plants. Fig. 3A shows the result of winter wheat stem samples (10 days after flowering). Fig. 3B shows the result of the leaf samples of transgenic *Yukihikari* containing introduced 6-SFT gene. 1K, 1-kestose; B, bifurcose; 2, 1 &6,1-kestopentaose; 4, 1,1,1 &6-kestohexaose; 6, 1,1,1,1 &6-kestoheptose; and 6K, 6-kestose. 1, 3, 5, and 7 indicate β(2→6)-linked fructans (levan) each differing in degree of polymerization.

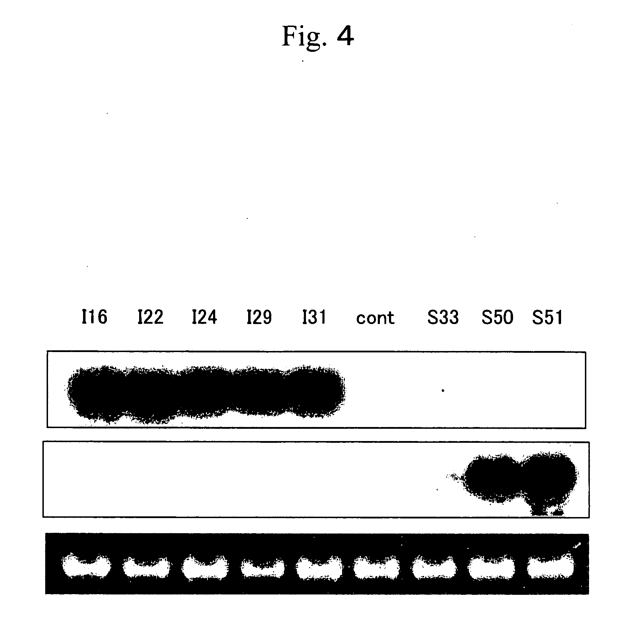

Fig. 4 shows a photograph showing the expression levels (at the mRNA level) of a 1-SST gene and a 6-SFT gene in transgenic rice plants containing introduced 1-SST gene and trasngenic rice plants containing introduced 6-SFT gene. I16, I16-2-1 line; 122, I22-1-1 line; I24, I24-6-1 line; I29, I29-5-1 line; I3 1, I31-10-1 line; cont, original cultivar *Yukihikari*; S33, S33-5-2 line; S50, S50-5-1 line; and S51, S51-2-1 line.

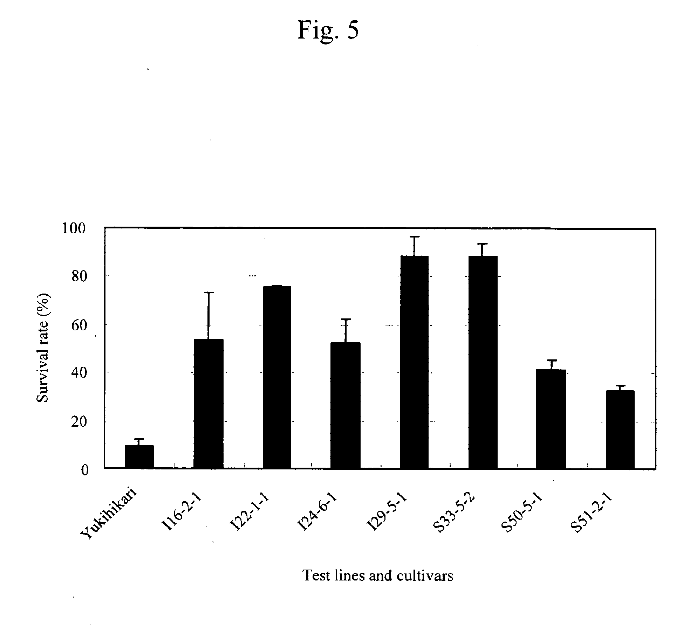

Fig. 5 shows seedling-stage chilling tolerance of each line of transgenic rice plants containing introduced 1-SST gene or 6-SFT gene.

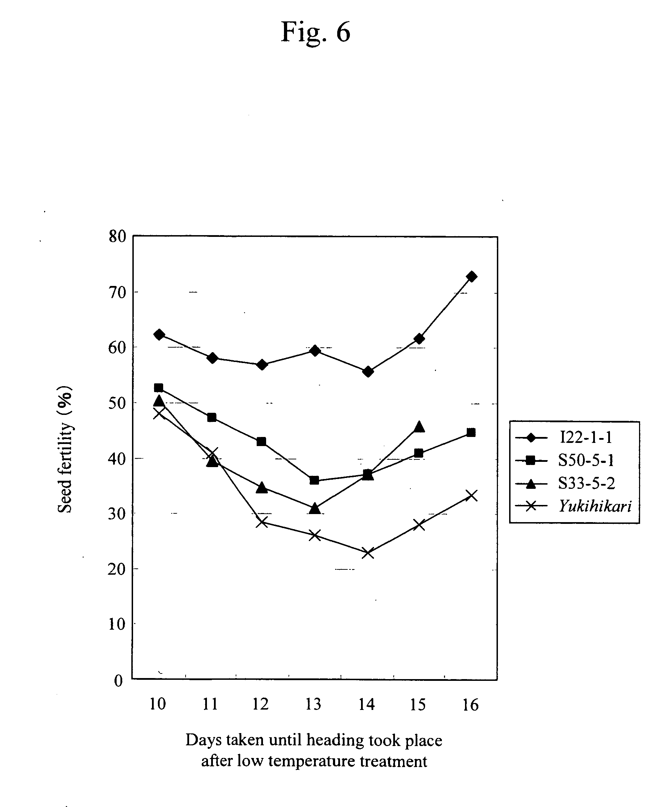

Fig. 6 shows booting-stage chilling tolerance of each line of transgenic rice plants containing introduced 1-SST gene or 6-SFT gene.

Best Mode for Carrying Out the Invention

Hereinafter, the present invention will be described in detail.

1. Transgenic rice plant of the present invention and its production

1) Transgenic rice plant

The transgenic rice plant of the present invention is a transformed rice plant that retains a gene-introduced fructosyltransferase gene, a sucrose:sucrose 1-fructosyltransferase (1-SST) gene or a sucrose:fructan 6-fructosyltransferase (6-SFT) gene within the cells.

In the transgenic rice plant of the present invention, the introduced 1-SST gene or 6-SFT gene is preferably incorporated within the chromosome of a host rice cell, but may also be retained extrachromosomally. When the 1-SST gene or the 6-SFT gene is incorporated within the chromosome of the transgenic rice plant, it is more preferable that such transgene is homozygous, but it may also be heterozygous. The transgenic rice plant of the present invention may have three or more copies of such transgene.

The term "transgenic rice plant" used herein means not only transgenic rice whole plants, but also rice cells expressing a transgene or retaining a transgene in a state of being capable of expressing a transgene, and rice organs (e.g., leaves, flowers, stems, roots, and grains (seeds)), rice tissue (e.g., epidermis, phloem, parenchyma, xylem, vascular bundle, palisade tissue, and spongy tissue), and cultured rice cells (e.g., callus), which contain such rice cells. Examples of the transgenic rice plant of the present invention include progeny of a transgenic rice whole plant regenerated from the rice cells into which the gene has been introduced or calli comprising such rice cells, and portions of such progeny.

2) Fructosyltransferase gene used in the present invention and its preparation

A 1-SST gene and a 6-SFT gene that are fructosyltransferase genes to be introduced into rice plants in the present invention may be isolated from various biological origins including plants, bacteria, and fungi. Examples of such biological origins preferably include, but are not limited to, overwintering organisms and organisms having cold tolerance (snow tolerance or freezing tolerance). The 1-SST gene and the 6-SFT gene are more preferably, but are not limited to, a 1-SST gene and a 6-SFT gene isolated from winter wheat *(Triticum aestivum* L.). Moreover, not only such 1-SST gene and 6-SFT gene isolated from an organism, but also mutated genes that comprise nucleotide sequences derived from the nucleotide sequences of the 1-SST gene and the 6-SFT gene by e.g., substitution or deletion of a nucleotide (s), but still encode fructosyltransferases can also be used in the present invention.

A specific example of preferred 1-SST gene in the present invention is a winter wheat 1-SST gene, which is a gene comprising the nucleotide sequence shown in SEQ ID NO: 1 and encoding sucrose:sucrose 1-fructosyltransferase comprising the amino acid sequence of SEQ ID NO: 2. An example of preferred 6-SFT gene in the present invention is a whinter wheat 6-SFT gene, which is a gene comprising the nucleotide sequence shown in SEQ ID NO: 3 and encoding sucrose:fructan 6-fructosyltransferase comprising the amino acid sequence of SEQ ID NO: 4.

Furthermore, the 1-SST gene or the 6-SFT gene according to the present invention may be a gene comprising DNA that hybridizes under stringent conditions to DNA having the nucleotide sequence shown in SEQ ID NO: 1 or 3 and encodes a protein having fructan synthetic activity.

The 1-SST gene or the 6-SFT gene according to the present invention may be a gene encoding a protein that comprises the amino acid sequence shown in SEQ ID NO: 2 or 4. Furthermore, the 1-SST gene or the 6-SFT gene according to the present invention may also be a gene encoding a protein that comprises an amino acid sequence derived from the amino acid sequence shown in SEQ ID NO: 2 or 4 by deletion, substitution, or addition of one or several (2 to 9 and preferably 2 to 5) amino acids and has fructan synthetic activity.

Alternatively, the 1-SST gene or the 6-SFT gene of the present invention may be a gene comprising a nucleotide sequence that exhibits at least 85% or more and preferably 90% or more identity with the nucleotide sequence shown in SEQ ID NO: 1 or 3 and encoding a protein that has fructan synthetic activity. Furthermore, the 1-SST gene or the 6-SFT gene of the present invention may be a gene encoding a protein that comprises an amino acid sequence exhibiting at least 60% or more, preferably 70% or more, and more preferably 85% or more identity with the amino acid sequence shown in SEQ ID NO: 2 or 4 and has fructan synthetic activity.

In the present invention, the term "stringent conditions" refers to conditions under which specific nucleic acids are hybridized. More specifically, such stringent conditions refer to reaction conditions in which the sodium salt concentration ranges from 15 mM to 750 mM, preferably 50 mM to 750 mM, and more preferably 300 mM to 750 mM, the temperature ranges from 25°C to 70°C and more preferably 55°C to 65°C, and the formamide concentration ranges from 0% to 50% and more preferably 35% to 45%. Furthermore, under stringent conditions, preferably post-hybridization filter-washing conditions comprise the sodium salt concentration ranging from 15 mM to 600 mM, preferably 50 mM to 600 mM, and more preferably 300 mM to 600 mM, and the temperature ranging from 50°C to 70°C, preferably 55°C to 70°C, and more preferably 60°C to 65°C.

The "gene" used herein can be DNA or RNA. Examples of the DNA include at least genomic DNA and cDNA. Examples of the RNA include mRNA and the like. The 1-SST gene and the 6-SFT gene of the present invention may also contain a untranslated region (UTR) sequence and so on, in addition to the open reading frame sequence of the 1-SST gene or the 6-SFT gene.

In addition, the fructan synthetic activity of the enzyme encoded by the 1-SST gene or the 6-SFT gene of the present invention is preferably sucrose:sucrose 1-fructosyl transferase activity in the case of the 1-SST gene and preferably sucrose:fructan 6-fructosyl transferase activity in the case of the 6-SFT gene.

The fructan synthetic activity of the enzyme (protein) encoded by the 1-SST gene or the 6-SFT gene of the present invention is a function of synthesizing a fructan with a longer chain using a sucrose and a fructan as substrates (Kawakami A. and Yoshida M., Biosci. Biotechnol. Biochem. (2002) 66 (11), pp. 2297-2305). Specifically, fructosyltransferase 1-SST mainly has activity of synthesizing 1-kestose (a trisaccharide fructan) by transfering a fructose derived from one of the two molecules of sucrose as substrates to the other sucrose via β2,1-linkage. Fructosyltransferase 1-SST also has activity of synthesizing fructan with a longer chain by transferring a fructose to the synthesized 1-kestose via β2,1-linkage. On the other hand, fructosyltransferase 6-SFT mainly has activity of generating, when two sucrose molecules are used as substrates, glucose and fructose by degrading the sucrose, while having activity of synthesizing 1-kestose or 6-kestose by transferring a fructose derived from one of the two molecules of sucroses to the other sucrose via β2,1- or β2,6-linkage. 6-SFT further has activity of synthesizing fructan with a longer chain by transfering a sucrose-derived fructose to a fructan via β2,6-linkage using a sucrose and a fructan as substrates.

The fructan synthetic activity of these fructosyltransferases can be confirmed according to Kawakami A. and Yoshida M., Biosci. Biotechnol. Biochem. (2002) 66 (11), pp. 2297-2305, for example, as described below. First, an isolated gene is inserted into a recombinant expression vector, yeast is transformed using the recombinant expression vector, protein expression from the recombinant vector is induced in the thus obtained transformant, and then the transgene-derived protein is purified and concentrated. The concentrated solution is used as a crude enzyme solution, followed by 2 hours of reaction at 10°C using sucrose as a substrate or sucrose and 1-kestose as substrates. The resultant is analyzed by a high performance anion-exchange chromatography (HPAEC) method using DX500 (DIONEX) and a Carbopac PA-1 column (DIONEX). As a result, when analysis is performed using a crude enzyme solution derived from yeast into which a 1-SST gene has been introduced and sucrose as a substrate, sucrose:sucrose 1-fructosyl transferase activity of the enzyme encoded by the 1-SST gene can be confirmed through detection of a peak indicating 1-kestose.

Meanwhile, when similar analysis is performed using a crude enzyme solution derived from yeast into which a 6-SFT gene has been introduced and sucrose as a substrate, the sucrose:fructan 6-fructosyltransferase activity of the enzyme encoded by the 6-SFT gene can be confirmed by detection of 1-kestose and 6-kestose peaks as well as fructose and glucose peaks indicating the degrading activity of the enzyme. When a sucrose and a 1-kestose are used as substrates, the sucrose:fructan 6-fructosyltransferase activity of the enzyme encoded by the 6-SFT gene can also be confirmed by detection of a bifurcose (a tetrasaccharide fructan having one fructose binding to a fructose as a constituent unit of sucrose via β2,1-linkage and another fructose binding to the same via β2,6-linkage).

DNA fragments containing the 1-SST gene or the 6-SFT gene of the present invention as described above can be obtained by persons skilled in the art according to a conventional technique from nucleic acids that are extracted from organisms having a 1-SST gene and a 6-SFT gene using known 1-SST gene and 6-SFT gene seqeunces.

In one embodiment, a cDNA clone containing a full-length 1-SST or 6-SFT gene can be isolated by PCR amplification using cDNA synthesized from mRNA extracted based on a conventional technique from fructan-accumulation tissue of a fructan-accumulation plant, as a template, and primers designed based on a highly conserved region among known fructosyltransferase genes to obtain a partial fragment of the 1-SST or 6-SFT gene, and then performing hybridization with a filter on which a cDNA library derived from the above cDNA has been blotted, using the partial fragment as a probe.

In another embodiment, a DNA fragment containing the fructosyltransferase genes 1-SST or 6-SFT according to the present invention can be obtained as a full-length cDNA by PCR amplification using mRNA extracted by a conventional technique from fructan-accumulation tissue of a fructan-accumulation plant, as a template, and primers designed to make it possible to amplify the full-length 1-SST gene or 6-SFT gene.

Furthermore, the nucleotide sequence of the thus obtained DNA fragment containing such fructosyltransferase gene 1-SST or 6-SFT can also be altered by a site-directed mutagenesis method or the like. For introduction of a mutation into DNA, known techniques such as the Kunkel method, the gapped duplex method, or any techniques based on those methods can be employed. For example, mutation may be introduced using a mutagenesis kit such as Mutant-Super Express Kit (TAKARA BIO INC.) or the LA PCR™ *in vitro* Mutagenesis Series Kit (TAKARA BIO INC.).

In a preferred example, a DNA fragment containing a winter wheat-derived 1-SST gene or 6-SFT gene can be obtained by PCR amplification using cDNA synthesized from mRNA extracted from a winter wheat plant exposed to low temperatures, as a template, and the following primer pairs designed for amplification of the full-length open reading frame.

Primer pair for amplification of 1-SST gene:

Primer Pri-1 (5'-atggattcgtctcgcgtc-3' [SEQ ID NO: 5])
Primer Pri-2 (5'-ctaatcgacgactaccaagtc-3' [SEQ ID NO: 6])

Primer pair for amplification of 6-SFT gene:

Primer Pri-3 (5'-atggggtcacacggcaag-3' [SEQ ID NO: 7])
Primer Pri-4 (5'-tcattgaacatacgagtgatc-3' [SEQ ID NO: 8])

The following composition can be used as the composition of a PCR reaction solution (20 μl) to be used for PCR amplification of the winter wheat-derived full-length 1-SST gene or 6-SFT gene, for example.

Table1: Composition of reaction buffer

| | |
|---|---|
| cDNA | 100 ng |
| Pri-1 or Pri-3 | 0.5 μM |
| Pri-2 or Pri-4 | 0.5 μM |
| dNTPs | 0.2 mM |
| MgCl$_2$ | 1.5 mM |
| ExTaq DNA polymerase (TAKARA BIO INC.) | 1 U |
| Total | 20 μl |

The PCR amplification can be performed under the following reaction conditions: 30 cycles of 94°C for 1 minute, 56°C for 1 minute, and 72°C for 2 minutes.

Such primers, PCR conditions, and the like preferred for preparation of a DNA fragment containing a winter wheat 1-SST gene or 6-SFT gene can be preferably employed by persons skilled in the art for other organisms (e.g., barley and perennial ryegrass) while adapting them for the organisms as appropriate.

It is useful to clone the thus obtained DNA fragment containing fructosyltransferase gene 1-SST or 6-SFT into a vector in advance. A DNA fragment containing a 1-SST gene or a 6-SFT gene may be ligated downstream of a plant overexpression promoter in a recombinant expression vector. In this case, for example, a DNA fragment containing a 1-SST gene or a 6-SFT gene is incorporated into such a vector by cleaving the DNA fragment with an appropriate restriction enzyme and then inserting in-frame and ligating the DNA fragment into an appropriate restriction enzyme site located downstream of an overexpression promoter in the vector. When Agrobacterium method is employed for gene transfer into rice plants, it is preferable to insert fructosyltransferase gene 1-SST or 6-SFT into a preferred vector for Agrobacterium method such as Agrobacterium-derived plasmid vectors (e.g., pBI101 or pBI121), expression vectors prepared based on such vector (e.g., pMLH7133), and binary vectors (pPZP2H-lac). For example, to construct a preferred vector for performing gene transfer by Agrobacterium method, a GUS gene under the control of a CaMV 35S promoter in pMLH7133 (Mitsuhara et al., 1996) can be substituted with the 1-SST gene or the 6-SFT gene of the present invention, so as to locate the 1-SST or 6-SFT gene under the control of the CaMV 35S promoter that is an overexpression (high-level expression) promoter. The thus constructed recombinant expression vector may further contain a cis element such as an enhancer, a splicing signal, a polyA addition signal, a selectable marker, a ribosome binding sequence (SD sequence), and the like. In addition, examples of a selectable marker include an ampicillin resistance gene, a neomycin resistance gene, and a hygromycin resistance gene.

In addition, it is preferable to verify the nucleotide sequence of the thus obtained DNA fragment containing fructosyltransferase gene 1-SST or 6-SFT via sequencing. The nucleotide sequence can be determined by a known technique such as Maxam-Gilbert chemical modification method and a dideoxynucleotide chain termination method. In general, the nucleotide sequence can be determined using an automated sequencing apparatus (e.g., DNA sequencer PRISM377XL, Applied Biosystems). Moreover, it is also preferable to verify the fructan synthetic activity of a protein that is encoded by the 1-SST gene or the 6-SFT gene contained in the thus obtained DNA fragment through the use of the above-mentioned transformation of yeast, or the like.

Molecular biological and biochemical experimental protocols employed in the present invention, such as mRNA preparation, cDNA preparation (RT-PCR), PCR, library construction, ligation into vectors, transformation of cells, DNA sequencing, primer synthesis, site-directed mutagenesis, and protein extraction can be performed according to methods as described in ordinary laboratory manuals. An example of such a laboratory manual is Sambrook et al., Molecular Cloning, A Laboratory Manual, 2001, Eds., Sambrook, J. & Russell, D. W., Cold Spring Harbor Laboratory Press.

3) Introduction of fructosyltransferase gene into rice plant

In the present invention, such fructosyltransferase gene 1-SST or 6-SFT obtained as described above can be introduced into rice *(Oryza sativa)* plants to produce transgenic rice plants.

Examples of preferred rice *(Oryza sativa)* cultivars used herein as host plants include, but are not limited to, *"Yukihikari," "Nihonbare," "Hoshinoyume," "Kirara397," "Yukimaru," "Honoka224," "Dontokoi," "Koshihikari," "Sasanishiki," "Hitomebore," "Akitakomachi," "Haenuki," "Kinuhikari," "Mutsuhomare," "Hinohikari," "Tsugaruotome," "Tsugaruroman," "Yumeakari," "Hanaechizen," "Yumetsukushi," "Hatsushimo," "Domannaka," " "Kakehashi," "Iwatekko," "Manamusume,." "Menkoina," "Chiyonishiki,"* and *"Fukumirai."* These rice cultivars can be obtained from NIAS Genebank (Genebank, the National Institute of Agrobiological Sciences, Japan), for example.

Examples of additional preferred rice cultivars include, but are not limited to, Wagwag (Philippines), Originario (Italy), Padano (Italy), Vialone Naro (Italy), Ribe (Italy), Baldo (Italy), Roma (Italy), Bomba (Spain), Makalioka (Colombia), IR 8 (Philippines), Taipei 309 (Taiwan), San You 63 (China), Liang You (China), Pei Kyu (China), King You 725 (China), II You 63 (China), Nan Keng (China), He Jiang 15 (China), Ewan 3 (China), Chu Chung (South Korea), Gyunggi (South Korea), Milyang 42 (South Korea), Milyang 30 (South Korea), Suweon 82 (South Korea), IRI 265 (South Korea), and Tep Trang (South Korea). These cultivars can be obtained from the NIAS Genebank (Genebank, the National Institute of Agrobiological Sciences, Japan), IRRI (International Rice Research Institute, Philippine), or the China National Rice Research Institute, for example.

Any plant transformation methods that are broadly used for plants can be used as a method for introducing a 1-SST gene or a 6-SFT gene into rice plants, including, but are not limited to, an Agrobacterium method, a particle gun method, an electroporation method, a polyethylene glycol (PEG) method, a microinjection method, and a protoplast fusion method. These plant transformation methods are described in detail in and can be performed with reference to ordinary textbooks such as "New Edition, Experimental Protocols for Model Plants, Genetic Techniques to Genomic Analysis" (under the supervision of Isao Shimamoto and Kiyotaka Okada, (2001) Shujunsha Co., Ltd.) and reports such as Hiei Y. et al., "Efficient transformation of rice (Oryza sativa L.) mediated by Agrobacterium and sequence analysis of the boundaries of the T-DNA," Plant J. (1994) 6, 271-282; Hayashimoto, A. et al., and "A polyethylene glycol-mediated protoplast transformation system for production of fertile transgenic rice plants." Plant Physiol. (1990) 93, 857-863.

When the Agrobacterium method is employed, a plant expression vector constructed by inserting fructosyltransferase gene 1-SST or 6-SFT into a vector suitable for the Agrobacterium method is introduced into appropriate Agrobacterium (e.g., *Agrobacterium tumefaciens)* as described above. Rice calli are then infected by inoculation of the bacterial strain, so that calli containing the transgenic rice cells can be obtained.

When the particle gun method, the electroporation method, or the like is employed, a 1-SST gene or a 6-SFT gene may be a PCR amplification fragment or a restriction enzyme-cleaved fragment containing the full-length gene or may also be a gene incorporated in a vector. As plant samples to be subjected to gene transfer, rice whole plants, rice organs, and rice tissue itself can also be used intact. Alternatively, sections of such samples and a protoplast prepared therefrom can also be used (Christou P, et al., Biotechnology 9: 957 (1991)). When such samples are subjected to the particle gun method, for example, a gene transfer apparatus (e.g., PDS-1000 (BIO-RAD)) is used according to the manufacturer's instructions. Specifically, transgenic rice cells can be obtained by bombarding gold particles coated with the fructosyltransferase gene 1-SST or 6-SFT into samples to introduce the gene into rice cells. Bombardment is generally performed at a pressure between approximately 450 psi and 2000 psi and at a distance approximately between 4 cm and 12 cm from the target.

Transgenic rice cells into which a 1-SST gene or a 6-SFT gene has been introduced or calli containing such transgenic cells can be cultured in selection medium according to a conventionally known plant tissue culture method, for example. Calli that have survived can be then cultured in redifferentiation medium (containing a plant hormone (e.g., auxin, cytokinin, gibberellin, abscisic acid, ethylene, or brassinolide) at an appropriate concentration) to regenerate transgenic rice whole plants that have been transformed with the 1-SST gene or the 6-SFT gene.

Whether or not fructosyltransferase gene 1-SST or 6-SFT is successfully introduced into rice cells can be confirmed using a PCR method, a Southern hybridization method, a Northern hybridization method, Western blot method, or the like. For example, total RNA is prepared from the leaves of the relevant transgenic rice plants, primers specific to fructosyltransferase gene 1-SST or 6-SFT are designed, and then PCR amplification is performed with the primers. Subsequently, the amplification product is subjected to agarose gel electrophoresis, polyacrylamide gel electrophoresis, capillary electrophoresis, or the like, followed by staining using ethidium bromide, SYBR Green liquid, or the like. The amplification product is then detected in the form of a single band, so that successful introduction of the 1-SST gene or the 6-SFT gene can be confirmed. Moreover, PCR is performed using 1-SST-gene- or 6-SFT-gene-specific primers labeled in advance with a fluorescent dye or the like, so that the PCR amplification product can also be detected. Furthermore, the amplification product can be also confirmed herein by a method involving binding a PCR amplification product to a solid phase such as a microplate and then detecting a fluorescence reaction, an enzyme reaction, or the like thereof.

It is also preferable to confirm that the introduced 1-SST gene or 6-SFT gene is expressed under general conditions (e.g., a temperature of 25°C) in the thus produced transgenic rice plants. For example, tissue sections are excised from transgenic rice plants that have grown for 10 days after seeding under general conditions. mRNA is then extracted from the samples of tissue sections by a conventional technique and then subjected to Northern blotting analysis using a probe specific to the 1-SST gene or the 6-SFT gene. Expression of the gene is then confirmed. Such a probe specific to a 1-SST gene or a 6-SFT gene can be designed by a conventional technique based on the nucleotide sequence of a 1-SST gene or a 6-SFT gene introduced. Alternatively, transgenic rice plants are grown for 10 to 14 days after seeding under general conditions and then tissue sections are excised. Soluble sugars are then extracted from the samples of tissue sections by a conventional technique and then analyzed by an HPLC method using Shodex KS802 and KS803 columns connected together or by the above-described HPAEC method, so that it can also be confirmed that fructan accumulation takes place within rice tissue.

Transgenic rice plants produced as described above, in which the fructosyltransferase gene of the present invention, the 1-SST gene or the 6-SFT gene, has been introduced, produce fructan (preferably constantly) within the rice cells, thereby exerting high-level chilling tolerance.

The present invention further relates to a method for producing a transgenic rice plant having enhanced chilling tolerance as described above and a method for enhancing chilling tolerance of a rice plant, which comprises introducing a 1-SST gene or a 6-SFT gene.

In addition, it is known that exposure of plants other than rice plants to cool temperatures during the early growth stage or pollen formation stage (corresponding to the rice booting stage) generally causes great damage to plant growth and seed or fruit-body production (Garham D. and Patterson B. D., Ann. Rev. Plant Physiol. (1982) 33: pp. 347-372). Therefore, the methods of the present invention can also be applied to plants other than rice plants, such as the other plants of the family Gramineae (e.g., corn *(Zea mays),* wheat *(Triticum aestivum* L.), barley *(Hordeum vulgare* L.), and rye *(Secale cereale)),* and any plants which are expected to have chilling tolerance, such as plants of the family Solanaceae, the family Leguminosae, the family Cucurbitaceae, or the like, thereby producing those plants with enhanced chilling tolerance, as in the case of rice.

## 2. Chilling tolerance of transgenic rice plant containing introduced fructosyltransferase gene 1-SST or 6-SFT

Transgenic rice plants containing the fructosyltransferase gene 1-SST or 6-SFT of the present invention have more enhanced chilling tolerance compared with that of rice plants of original cultivars (or wild type) into which neither the 1-SST gene nor the 6-SFT gene is introduced.

Rice plants are summer crop plants. Generally in the case of single cropping in Japan, planting (seeding) is performed from about April through May, followed by harvesting from late summer to autumn. After germination, rice plants experience growth stages including the seedling stage, the tillering stage, the panicle formation stage, the booting stage (pollen formation stage), the heading stage, the flowering stage, the ripening stage, and the like, and then they finally produce mature seeds. Rice plants are originally tropical crop plants. Hence, when rice plants encounter cool temperatures during their growth, they will be damaged in various ways and the resulting yields will be significantly decreased (i.e., the occurrence of cool weather damage). For example, when rice plants encounter temperature conditions of 12°C or less during the seedling stage, 19°C or less during the booting stage, and 15°C or less during the ripening stage, there is a high possibility that cool weather damage would take place. Specific types of damage are caused by cool temperatures vary depending on the growth stages during which rice plants are exposed to cool temperatures. For example, exposure to cool temperatures throughout the growth stages ranging from the seedling stage (early growth stage) to the ripening stage causes growth delay. As a result, not only is poor ripening of exposed plants caused by chilling at early autum, but occurrences of diseases such as rice blast also increase. In the meantime, exposure to cool temperatures during stages ranging from the booting stage to the flowering stage causes unfertilization that lowers the seed fertilily, and thus it often results in production of unfilled spikelets and fall of spikelets. Exposure to cool temperatures during the seedling stage, the booting stage, and the flowering stage tends to cause particularly severe cool weather damage.

In the transgenic rice plant of the present invention, tolerance to cool temperatures at each growth stage as described above is enhanced. In particular, enhanced seedling-stage chilling tolerance and/or enhanced booting-stage chilling tolerance are found in the transgenic rice plant of the present invention. Seedling-stage chilling tolerance means a tolerance to cool temperatures to be exposed during the seedling stage that corresponds to the early growth stage after germination. Booting-stage chilling tolerance means a tolerance to cool temperatures to be exposed during the booting stage that corresponds to stages including meiosis of reproductive cells such as pollen and the following maturation stages. Accordingly, in a preferred embodiment, the damage that the transgenic rice plant of the present invention will experience when it is exposed to cool temperatures during the seedling stage or the booting stage can be significantly alleviated.

In the present invention, the term "chilling tolerance" refers to ability to alleviate chilling damage; that is, the ability of rice plants to grow while alleviating or without experiencing any damage when they are exposed to cool temperatures during any growth stages. In the present invention, the term "cool temperatures" means low temperatures within a positive temperature range, and more specifically, it means temperature conditions in which the daily mean temperature is greater than 0°C and less than 20°C (in the Description, also conveniently denoted as "0°C to 20°C"). "Chilling damage" that can be alleviated by the use of the transgenic rice plant of the present invention may be any damage that rice plants exposed to cool temperatures can experience. Examples of such "chilling damage" include, but are not limited to, delayed growth, poor growth, wilt, reduced numbers of stems during the tillering stage, delayed panicle formation, reduced numbers of spikelets, delayed heading, delayed flowering, unfertilization, failure in flowering, and poor ripening; and as a result thereof, decreased survival rate, decreased seed fertility, decreased yield and the like. Regarding detailed information concerning chilling damage of rice plants, Science of Rice Plant Physiology, Vol. 2, Physiology, pp. 769-812, ISBN 4-540-94051-1 can be referred to.

The enhanced chilling tolerance in the transgenic rice plant of the present invention can be confirmed by the fact that the degree of chilling damage is alleviated compared with that in the cases of rice plants of original cultivars (or wild type).

The present invention also relates to the use of the transgenic rice plant of the present invention for cultivation under cool temperature conditions. The present invention further relates to the use of the transgenic rice plant of the present invention for alleviating chilling damage in rice plant cultivation, and especially, chilling damage that takes place during the rice seedling stage and/or booting stage.

The excellent seedling-stage chilling tolerance and booting-stage chilling tolerance of the transgenic rice plant of the present invention will be described in detail below.

Seedling-stage chilling tolerance

The rice seedling stage is a growth stage that begins after germination and ends at around the 4th leaf stage. In the case of single cropping in a year in Japan, the seedling stage corresponds to the period between roughly mid-May and early June, although it varies depending on cultivation methods, weather of the relevant year, and the like. Examples of damage due to cool temperatures at the seedling stage include death and the resulting decreased survival rate, poor growth, delayed growth, and development of wilt. Particularly in the case of direct rice seeding cultivation, it is known that satisfactory or unsatisfactory of the growth of rice plants at the seedling stage has a significant influence on their subsequent growth.

"Seedling-stage chilling tolerance" in the present invention can be evaluated based on, but as a non-limiting example, the survival rate of rice plants that have been exposed during the seedling stage to cool temperatures such as a daily mean temperature of preferably more than 0°C and 12°C or less (0°C to 12°C) and more preferably more than 0°C and 5°C or less (0°C to 5°C). Such evaluation of seedling-stage chilling tolerance can be performed by a standard chilling tolerance test and is particularly preferably performed by the following procedures.

First, rice seeds are seeded and allowed to germinate, and then grown under temperature conditions of 25°C/ 19°C (day/night) (a cycle of 25°C during daytime (15 hours) and 19°C during nighttime (9 hours)). Subsequently, seedlings (leaf age between 2.5 and 3.5 and plant length of around 10 cm) on day 10 after seeding are treated under temperature conditions of 5°C (day and night) for 14 days. The rice plants are then grown under the above temperature conditions of 25°C/19°C again. The survival rate of the rice plants after 7 days of completion of cool temperature treatment is examined. As conditions other than such temperatures and the like, general conditions that are employed in experimental protocols for rice cultivation are employed herein. Survival rate is determined as follows. Individual plants that develop new leaves (restart of growth) within 7 days after completion of cool temperature treatment are determined to be surviving plants. Individual plants that do not develop any new leaves (do not restart their growth) are determined to be dead plants. Survival rate (%) is then calculated as follows:

$$\text{Survival rate (\%)} = \text{(number of individual plants that underwent new leaf development)} / \text{(number of all the tested plants)} \times 100.$$

When the survival rate of transgenic rice plants subjected to cool temperature treatment during their seedling stage is higher than that of rice plants of the original cultivar, it can be determined that seedling-stage chilling tolerance is enhanced in the transgenic rice plants. In the transgenic rice plant of the present invention, preferably such survival rate that is calculated as described above is significantly higher than that in the case of rice plants of the original cultivar. The survival rate of the transgenic rice plant of the present invention preferably ranges from 30% to 100% and more preferably ranges from 50% to 100%.

Booting-stage (pollen formation stage) chilling tolerance

The rice booting stage is a period approximately 16 to 10 days before heading, during which pollen formation and development take place. In the case of single cropping in Japan, the booting stage corresponds to a period between around the mid-July and early August, although it varies depending on cultivation methods, weather of the relevant year, and the like. Examples of damage due to cool temperatures during the booting stage include unfertilization and the resulting male sterility (lowered seed fertility), delayed heading, and development of leaf sheath browning. The rice booting stage is a period that has significant effects on the following seed formation. Exposure to cool temperatures during the booting stage results in greatly decreased number of seed formation because of suppressed pollen formation.

"Booting-stage chilling tolerance" in the present invention can be evaluated based on, but as a non-limiting example, the seed fertility of rice plants that have been exposed during the booting stage to cool temperatures such as a daily mean temperature of more than 0°C and less than 20°C and preferably 12°C or more and 18°C or less. Such evaluation of booting-stage chilling tolerance can be performed by a standard chilling tolerance test and is particularly preferably performed by the following procedures.

First, rice seeds are seeded and allowed to germinate, and then grown under temperature conditions of 26°C/ 20°C (day/night) (a cycle of 26°C during daytime (15 hours) and 20°C during nighttime (9 hours)). Subsequently, rice plants at a growth stage 16 days to 10 days before the heading stage (in the case of *Yukihikari,* leaf age between 10 and 11 and auricle-to-auricle length between minus 4 cm and 0 cm) are grown for 4 days under temperature conditions of 12°C (day and night). The rice plants are then grown under the above temperature conditions of 26°C/20°C (day/ night) again, to allow them to undergo heading, flowering, and then seed formation. The seed fertility is then calculated. As conditions other than such temperatures and the like, general conditions that are employed in experimental protocols for rice cultivation are employed herein. For all spikelets that are collected from panicles in which 90% or more of spikelets of the whole panicles is yellowed, the number of all the spikelets (total number of spikelets) and the number of ripened seeds (number of spikelets containing seeds (rice content)) are counted. The seed fertility (%) is then calculated as follows:

$$\text{Seed fertility (\%)} = \text{(number of ripened seeds)} / \text{(number of all spikelets)} \times 100.$$

When the seed fertility rate found throughout a given line of the transgenic rice plants of the present invention subjected to cool temperature treatment during their booting stage or particularly during their booting stage 16 days before heading are higher than rice plants of the original cultivar, it can be determined that the booting-stage chilling tolerance is enhanced in such line of the transgenic rice plants. In the transgenic rice plant of the present invention, preferably the average seed fertility of each line that is calculated as described above is significantly higher than that in the case of rice plants of the original cultivar. The seed fertility rate of the transgenic rice plant of the present invention preferably ranges from 40% to 100% and more preferably ranges from 50% to 80%, when the plant is subjected to cool temperature treatment 16 days before heading.

Examples

The present invention is further described with reference to the following examples and drawings. However, the present invention is not limited by these examples.

Reference example 1: Isolation of fructosyltransferase genes

Obtainment of fructosyltransferase genes 1-SST and 6-SFT in the Reference example was performed according to the method of Kawakami A. and Yoshida M., Biosci. Biotechnol. Biochem. (2002) 66 (11), pp. 2297-2305.

1) Preparation of cDNA and cDNA library from winter wheat line PI 173438

mRNA was extracted by a conventional technique from the crown portions of the wheat plant *(Triticum aestivum* L.) line PI173438 obtained by seeding in late September in trays and then growing under natural conditions until November 22. cDNA was synthesized using 5 $\mu$g of the thus obtained mRNA and a cDNA synthesis kit (STRATAGENE). Furthermore, the synthesized cDNA was inserted into $\lambda$ZAP expression vector (STRATAGENE) to generate a cDNA library.

2) Isolation of 1-SST and 6-SFT partial gene fragments from the cDNA library

PCR was performed using cDNA synthesized as described above as a template and the following forward and reverse primers:

- Forward primer: 5' A-T-G-A-A-T/C-G-A-T/C-C-C-N-A-A-T/C-G-G 3' (SEQ ID NO: 9)
- Reverse primer: 5' C-C-N-G-T-N-G-C-A/G-T-T-A/G-T-T-A/G-A-A 3' (SEQ ID NO: 10).

These forward and reverse primers were designed on a highly conserved region among the nucleotide sequences of known fructosyltransferase genes (e.g., Genbank accession Nos. X83233, AJ006066, Y07838, AF492836, AJ567377, AJ009756, and Y09662). PCR was performed in a reaction system of 50 $\mu$l. The reaction solution was prepared by mixing 1 $\mu$l of ExTaq DNA polymerase (TAKARA BIO INC.; 5 units/$\mu$l), 5 $\mu$l of 10 x Polymerase buffer with MgCl$_2$, 5 $\mu$l of dNTPs solution (10 mM), 2 $\mu$l each of the above primers (12 $\mu$M), and approximately 10 ng of cDNA synthesized above, and adjusting to a total volume of 50 $\mu$l with distilled water. The PCR reaction conditions and number of cycles used are as listed in Table 2 below.

Table 2

| Reaction step | Reaction temperature | Reaction time | Number of cycles |
|---|---|---|---|
| Denaturation | 94°C | 1 minute | 1 cycle |
| Denaturation | 94°C | 1 minute | 30 cycles |
| Annealing | 50°C | 1 minute | |
| Elongation reaction | 72°C | 2 minutes | |
| Elongation reaction | 72°C | 2 minutes | 1 cycle |

After PCR, PCR products were confirmed by electrophoresis. Nucleic acid fragments of the expected length (around 1500 bp) were amplified. The thus obtained PCR fragments were cloned by a conventional technique and positive clones were obtained. The DNA insertion fragments contained in these positive clones were sequenced with a DYEnamic ET Terminal Cycle Sequencing Kit (Amersham Biosciences) and a DNA sequencer (PRISM 377XL, Applied Biosystems), and then subjected to comparison analysis with the above-described known fructosyltransferase genes. As a result, several DNA insertion fragments having 50% or more nucleotide sequence homology with known fructosyltransferase genes were found. These DNA fragments were considered to be the partial fragments of the wheat fructosyltransferase genes.

3) Isolation of cDNA containing full-length fructosyltransferase genes and determination of nucleotide sequences

Hybridization assay was performed by a conventional technique for the cDNA library obtained in 1) above, using the above fructosyltransferase gene partial fragments labeled with isotopes as probes. The DNA insertion fragments contained in the obtained positive clones were sequenced using a DYEnamic ET Terminal Cycle Sequencing Kit (Amersham Biosciences) and a DNA sequencer (PRISM 377XL, Applied Biosystems). As a result of analysis of the thus obtained nucleotide sequences, a clone having an insert DNA fragment comprising the nucleotide sequence (SEQ ID NO: 1) of 1989 bp encoding 662 amino acids, which have 72% amino acid sequence homology with that of perennial ryegrass 1-SST gene (Genbank accession No. AJ297369) was found. Similarly, a clone having an insert DNA fragment comprising the nucleotide sequence (SEQ ID NO: 3) of 1851 bp encoding 616 amino acids, which have 93% amino acid sequence homology with that of barley 6-SFT gene (Genbank accession No. X83233) was also found. Hence, these DNA insertion fragments were considered to be cDNAs containing different wheat fructosyltransferase genes, respectively.

4) Functional analysis of proteins encoded by isolated wheat fructosyltransferase genes

From the positive clones each containing one of the two types of full-length wheat fructosyltransferase gene obtained in 3) above, DNA fragments each containing either one of two open reading frames (the nucleotide sequence of SEQ ID NO: 1 or 3) were prepared. Each of the DNA fragments was cloned into an expression vector pPICZ$\alpha$B (Invitrogen, EasySelect Pichia Expression Kit). The vector was then introduced into yeast *(Pichia pastoris)* by electroporation in a conventional manner. The expression vector pPICZ$\alpha$B used herein contained the AOX1 promoter sequence for inducing the expression of a gene cloned into the vector specifically in response to methanol in yeast liquid medium, and the sequence encoding a secretion factor, $\alpha$ factor, that is required for secretion of a recombinant protein expressed outside of yeast cells; that is, in a culture medium.

Yeast transformed with the expression vectors, pPICZ$\alpha$B, containing each of the above DNA fragments was cultured for 4 days in liquid medium containing methanol as a carbon source. The culture medium was separated from the yeast cells and then concentrated approximately 20-fold with an ultrafiltration filter. The concentrated medium (crude enzyme solution) was mixed with sucrose or 1-kestose as a substrate, followed by 8 hours of reaction at 4°C. The reaction products were analyzed by the HPAEC method to examine the enzyme activity of the recombinant protein to be contained in the culture mediums. As a result, in the culture medium of the yeast into which the expression vector carrying a DNA fragment containing the nucleotide sequence of SEQ ID NO: 1 had been introduced, activity of synthesizing 1-kestose (a trisaccharide in which one fructose unit is transferred to a sucrose via $\beta$2,1-linkage) was detected when sucrose had been used as the sole substrate; while, activity of synthesizing 1,1-nystose (a tetrasaccharide) was detected at a level that was significantly lower than that of activity of synthesizing trisaccharide when 1-kestose is used as the sole substrate to the reaction. Therefore, it was demonstrated that the DNA fragment encoded an enzyme having sucrose: sucrose 1-fructosyltransferase activity. Hence, it was revealed that the gene contained in the DNA fragment was the wheat 1-SST gene.

Moreover, in the culture medium of the yeast into which the expression vector carrying a DNA fragment containing the nucleotide sequence of SEQ ID NO: 3 had been introduced, activity of synthesizing 1-kestose, activity of synthesizing 6-kestose (a trisaccharide in which one fructose is transferred to a sucrose via $\beta$2,6-linkage), and activity of synthesizing a fructan of tetra- or higher saccharide were detected when sucrose was added as the sole substrate; while, almost no activity of synthesizing a fructan of tetra- or higher saccharide was detected when 1-kestose is added as the sole substrate to the reaction. Therefore, it was demonstrated that the DNA fragment encoded an enzyme having sucrose:fructan 6-fructosyltransferase activity. Hence, it was revealed that the gene contained in the DNA fragment was the wheat 6-SFT gene.

As described above, isolation of the wheat 1-SST gene (SEQ ID NO: 1) and that of the 6-SFT gene (SEQ ID NO: 3) were confirmed.

Example 1: Transformation of rice plants via introduction of fructosyltransferase gene 1-SST or 6-SFT and regeneration of rice whole plants

As described below, the fructosyltransferase genes 1-SST and 6-SFT isolated in Reference example 1 were each inserted downstream of a CaMV 35S promoter in a Ti-based vector; pMLH7133 (Mitsuhara 1. et al., (1996) Efficient promoter cassettes for enhanced expression of foreign genes in dicotyledonous and monocotyledonous plant. Plant Cell Physiol. 37: 49-59) in the sense strand direction.

First, the full-length fructosyltransferase gene 1-SST cDNA was prepared by PCR amplification using the 1-SST-cDNA-containing clone obtained in Reference example 1 as a template, a primer 5'-ttggatccattatggattcgtctcgcgtc-3' (the underlined portion is a *Bam*H I site; SEQ ID NO: 11), and a primer 5'-aagagctcctaatcgacgactaccaagtc-3' (the underlined portion is a *Sac* I site; SEQ ID NO: 12) Moreover, the full-length fructosyltransferase gene 6-SFT cDNA was obtained by PCR amplification using the 6-SFT-cDNA-containing clone obtained in Reference example 1 as a template, a primer 5'-ctggatccaagatggggtcacacggcaag-3' (the underlined portion is a *Bam*H I site: SEQ ID NO: 13), and a primer 5'-ccg_a.gctctcattgaacatacgagtgatc-3' (the underlined portion is a *Sac* I site; SEQ ID NO: 14). These both amplification fragments were each treated with *Bam*H I and *Sac* I, thereby preparing *Bam*H I-*Sac* I fragments. The thus prepared 1-SST-gene-containing *Bam*H I-*Sac* I fragment or 6-SFT-gene-containing *Bam*H I-*Sac* I fragment was ligated to a pMLH7133 vector cleaved using *Bam*H I and *Sac* I in the sense strand direction. The thus obtained nucleic acid constructs were introduced into rice *(Oryza sativa* L. cv, *Yukihikari; "Yukihikari,")* calli via Agrobacterium method according to previously reported protocols (Hiei Y., Ohta S., Komari T., kumashiro T. (1994) "Efficient transformation of rice (Oryza sativa L.) mediated by Agrobacterium and sequence analysis of the boundaries of the T-DNA. " Plant J. 6: 271-282). To select the thus transformed calli, the calli subjected to the gene transfer were transplanted in MSRE medium and then selected based on hygromycin resistance. The selected calli were further transplanted in redifferentiation medium (N6SE) and thereby allowed to redifferentiate in a light condition. Next, the resulting redifferentiated individuals were placed on a test medium (MSHF) and then tested for hygromycin resistance. The individuals that had rooted and grown normally on the medium were allowed to be habituated and then transplanted in pots, thereby being further regenerated into rice whole plants. Crossing via self-pollination with the thus regenerated whole plants (To generation) were performed until $T_2$ generation plants were obtained. The compositions of culture media used in the above experiments are as shown below.

Table 3: MSRE medium (pH5.8)

| MS inorganic salt | |
|---|---|
| MS vitamin | |
| 30 g/l | sucrose |
| 30 g/l | sorbitol |
| 2 mg/l | casamino acid |
| 1 mg/l | NAA |
| 2 mg/l | BAP |
| 250 mg/l | carbenicillin |
| 50 mg/l | hygromycin |
| 4 g/l | Gelrite |

Table 4: N6SE medium (pH 5.8)

| N6 inorganic salt | |
|---|---|
| N6 vitamin | |
| 30 g/l | sucrose |
| 2 mg/l | 2,4-D |
| 2 g/l | Gelrite |
| 500 mg/l | carbenicillin |
| 50 mg/l | hygromycin |

Table 5: MSHF medium (pH 5.8)

| MS inorganic salt | |
|---|---|
| MS vitamin | |
| 30 g/l | sucrose |
| 50 mg/l | hygromycin |
| 8 g | agar |

Subsequently, shoots were each excised from the thus obtained To generation, $T_1$ generation, and $T_2$ generation plants. Genomic DNA was extracted from the shoots by a conventional technique. PCR screening was performed using the genomic DNA as a template, a primer (5'-ggtaagtaattgctactggtatcacttg-3'; SEQ ID NO: 15) specific to a first intron sequence of a soybean phaseolin gene contained in the promoter region of vector pMLH7133, and a primer (5'-ggagcccgacggggtgtactg-3'; SEQ ID NO: 16) specific to 1-SST cDNA or a primer (5'-ctcaacggcggaggcgtt-3'; SEQ ID NO: 17) specific to 6-SFT cDNA. Thus, homozygous lines were selected. As a result, the rice plant lines having fructosyltransferase gene 1-SST homozygously, I16-2-1, I22-1-1, 124-6-1, I29-5-1, and 131-10-1 lines were obtained. Furthermore, the rice plant lines having fructosyltransferase gene 6-SFT homozygously, S33-5-2, S50-5-1, and S51-2-1 lines were obtained.

Example 2: Expression analysis of transgenic rice plants containing the introduced fructosyltransferase genes

For the transgenic rice lines having homozygous fructosyltransferase genes ($T_2$ generation; original cultivar

*"Yukihikari")* obtained in Example 1, the amounts of fructan accumulated within tissue and fructosyltransferase gene expression when the fructosyltransferase genes had been over-expressed under the control of the CaMV 35S promoter were analyzed as described below.

1) Analysis of accumulated fructans and amounts thereof

Leaf tissue was obtained from each of all lines of the transgenic rice plants containing the introduced fructosyltransferase gene 1-SST or 6-SFT as obtained in Example 1, and control rice plants into which no fructosyltransferase gene had been introduced (i.e., original cultivar *"Yukihikari").* The one gram of such leaf tissue was finely cut and then boiled in 10 ml of hot water (containing 1 mg/ml propylene glycol as an internal standard) for 1 hour. The extract solution was filtered using a 0.45 $\mu$m filter. Each sugar content in the filtrate was analyzed by HPLC using RI detection and Shodex KS802 and KS803 columns (both available from Showa Denko KK., Tokyo, Japan) connected together under the condition at a column temperature of 50°C and an eluting rate of 0.8 ml/min using water as a mobile phase. Fructose content, glucose content, sucrose content, and fructan content were separately measured. With the analysis, no fructan was detected in the non-transgenic rice plants without the fructosyltransferase gene. However, fructan accumulation was detected in both of the transgenic rice plants containing the introduced fructosyltransferase genes, either 1-SST or 6-SFT.

The structure of accumulated fructan was analyzed by the HPAEC (High Performance Anion-Exchange Chromatography) method using DX500 (DIONEX). For this measurement, Carbopac PA1 (DIONEX) was used as a column and a Pulsed Amperometric Detector ED40 (DIONEX) was used as a detector. The column temperature was room temperature and 150 mM sodium hydroxide was used as an eluting solution. The components in the above filtrates were eluted with a concentration gradient of sodium acetate (25 mM to 500 mM, 40 minutes). The flow rate employed herein was 1 ml/min. The chromatogram obtained by this measurement for samples derived from the transgenic rice plants containing the introduced fructosyltransferase gene 1-SST was compared with that of control samples derived from the original cultivar *Yukihikari* and that of samples derived from Jerusalem artichoke tubers, which were obtained by the same measurement method. Fig. 1 shows the results.

It is known that Jerusalem artichoke accumulates fructan in which fructoses are polymerized to sucrose via $\beta$2,1-linkage in its tubers (Shiomi, N., New Phytologist (1993) vol. 123, pp. 263-270). Chromatogram (A) in Fig. 1 shows several peaks indicating fructans differing in degree of polymerization, which were accumulated in Jerusalem artichoke tubers. Corresponding to those peaks, it was shown in the case of samples derived from transgenic rice plants containing the introduced 1-SST gene that 1-kestose (a trisaccharide fructan in which another fructose is transferred via $\beta$2,1-linkage to a fructose as a constituent unit of sucrose) and fructans with a degree of polymerization ranging from 4 to 7 in which other fructose or fructoses are transferred via $\beta$2,1-linkage to 1-kestose had been accumulated (Fig. 1 (B)). Meanwhile, in the control rice plants of the original cultivar *Yukihikari,* sucrose peaks were detected, however, peaks corresponding to the fructans having different degrees of polymerization as descrived above were not detected. Therefore, it could be confirmed that the 1-SST enzyme capable of generating fructans each of which contains $\beta$2,1-linked fructose (s) was accumulated in transgenic rice plants containing the introduced 1-SST gene.

Furthermore, samples derived from the transgenic rice plants containing the introduced 1-SST gene, samples derived from the transgenic rice plants containing the introduced 6-SFT gene, and control samples derived from the original cultivar *Yukihikari* were compared among their chromatogram obtained by the above measurements. Fig. 2 shows the results. As shown in Fig. 2, in the samples derived from the transgenic rice plants containing the introduced 6-SFT gene, different soluble sugar peaks from those of the transgenic rice plants containing the introduced 1-SST gene were detected. Accordingly, it was demonstrated that sugars accumulated in the transgenic rice plants containing the introduced 6-SFT gene differed from those in the transgenic rice plants containing the introduced 1-SST gene.

Subsequently, the chromatogram of samples derived from the transgenic rice plants containing the 6-SFT gene was compared with the chromatogram obtained by the same measurement for samples that had been extracted in a manner similar to the above from the stems of winter wheat plants (10 days after flowering) that was an isolation source of the 6-SFT gene. Fig. 3 shows the results. In addition, it is known that many of fructans produced by winter wheat plants are fructans having $\beta$2,6-linked fructoses. The chromatogram in the upper row in Fig. 3 shows many peaks indicating fructans with various degrees of polymerization having $\beta$2,6-linkage, which were accumulated in the winter wheat stems. Corresponding to the many peaks, it was shown in the case of samples derived from transgenic rice plants containing the introduced 6-SFT gene that peaks of fructans in which other fructose or fructoses are transferred via $\beta$2,6-linkage to a fructose as a constituent unit of sucrose (see, 6K, 1, 3, 5, and 7 shown in Fig. 3(C)) as well as peaks of fructans in which other fructose or fructoses are transferred via $\beta$2,6-linkage to a fructose as a constituent unit bound to the glucose of 1-kestose (a trisaccharide; 1K shown in Fig. 3(C)) (see, B, 2, 4, and 6 shown in Fig. 3 (C)) were detected. Therefore, it could be confirmed that 6-SFT capable of producing fructans each of which that contains $\beta$2,6-linked fructose (s) was accumulated in the transgenic rice plants containing the introduced 6-SFT gene.

2) Northern blot analysis

Northern blot analysis was performed as described below for expression of the 1-SST gene and the 6-SFT gene in the transgenic rice plants containing the introduced 1-SST gene and the transgenic rice plants containing the introduced 6-SFT gene obtained in Example 1, respectively.

First, a probe for detection of 1-SST mRNA in this analysis was prepared by PCR amplification from the the full-length 1-SST cDNA-containing vector as obtained in Reference example 1 as a template, using primers 5'-gccaacgcgttcccgtggagc-3' (SEQ ID NO: 18) and 5'-atcgacgactaccaagtcatcatctgtgt-3' (SEQ ID NO: 19), and ExTaq polymerase (TAKARA BIO INC.). A probe for detection of 6-SFT mRNA was prepared by PCR amplification from the full-length 6-SFT cDNA-containing vector as obtained in Reference example 1 as a template, using primers 5'-gcgggcgggttcccgtggagcaa-3' (SEQ ID NO: 20) and 5'-ttgaacatacgagtgatcgtccatattggag-3' (SEQ ID NO: 21), and ExTaq polymerase (TAKARA BIO INC.). These amplification fragments were subjected to gel purification. The fragments are then each labeled with $^{32}$p-dCTP by the random hexamer priming method (BcaBest™ Labeling Kit; TAKARA BIO INC.) and then used as probes.

The transgenic rice plants containing the introduced 1-SST gene (I16-2-1 line, I22-1-1 line, I24-6-1 line, I29-5-1 line, and I31-10-1 line), the transgenic rice plants containing the introduced 6-SFT gene (S33-5-2 line, S50-5-1 line, and S51-2-1 line), and control rice plants of the original cultivar *Yukihikari* were seeded and grown at 25°C/19°C (day/night) under an artificlly controlled environment. On day 10 after the start of growing, RNAs were extracted by a conventional technique from portions above the ground of seedling plants of the transgenic rice plants and the control rice plants.

Each of the thus prepared total RNA was subjected to 1.2% agarose gel electrophoresis and then transferred onto nylon filters. The filters were then each subjected to pre-hybridization for 1 hour in a hybridization buffer (50% formamide, 5 x SSPE, 5 x Denhardt's solution, 0.5% SDS, and 0.2 mg/ml purified salmon sperm DNA). Subsequently, the probes prepared as described above were each added, followed by hybridization for 12 hours or more at 42°C. The filter was then washed twice with 2 x SSC and 0.1% SDS for 15 minutes at room temperature and then washed twice with 0.2 x SSC and 0.1% SDS at 65°C for 30 minutes. The washed filter was exposed to X-ray film for visualization.

Fig. 4 shows the results of the above Northern blot analysis. As shown in Fig. 4, expression of 1-SST could be found at the genetic level in all the transgenic rice plant lines containing the introduced 1-SST gene. Similarly, expression of 6-SFT could be also found at the genetic level in all the transgenic rice plant lines containing the introduced 6-SFT gene. In the control rice plants of the original cultivar *Yukihikari,* no expression was detected for both 1-SST gene and 6-SFT gene.

Example 3: Seedling-stage chilling tolerance of transgenic rice plants containing introduced fructosyltransferase genes

The four lines of transgenic rice plant containing the introduced 1-SST gene (I16-2-1 line, I22-1-1 line, I24-6-1 line, and I29-5-1 line), the three lines of transgenic rice plant containing the introduced 6-SFT gene (S33-5-2 line, S50-5-1 line, and S51-2-1 line), and original cultivar *"Yukihikari"* plants were seeded and grown at temperatures of 25°C/19°C (day/night) (a cycle of 25°C during daytime (15 hours) and 19°C during nighttime (9 hours)) under an artificially controlled environment.

On day 10 after seeding, rice seedlings (20 individual plants per line were used in the experiment in duplicate) were subjected to low temperature treatment at a temperature of 5°C (day and night) for 14 days. The rice seedlings were then grown under the above conditions of temperatures 25°C/19°C (day/night) again. The survival rates were examined after 14 days of completion of the low temperature treatment.

As a result, the survival rates of the transgenic rice lines containing the introduced 1-SST gene were 53.7% for the I16-2-1 line, 75.8% for the I22-1-1 line, 52.1% for the I24-6-1 line, and 88.1% for the 129-5-1 line. The survival rates of the transgenic rice lines containing the introduced 6-SFT gene were 88.4% for the S33-5-2 line, 41.2% for the S50-5-1 line, and 32.3% for the S51-2-1 line (Fig. 5). The survival rate of the original cultivar *"Yukihikari"* plants was 9.3% (Fig. 5). Thus, it was demonstrated that transgenic rice plants containing the introduced 1-SST gene or 6-SFT gene had high tolerance to cool temperatures to which they were exposed during their seedling stage.

Example 4: Booting-stage chilling tolerance of transgenic rice plants containing introduced fructosyltransferase genes

The transgenic rice plant line containing the introduced 1-SST gene (the line I22-1-1), the transgenic rice plant lines containing the introduced 6-SFT gene (S33-5-2 and S50-5-1), and the original cultivar *"Yukihikari"* plants were seeded and grown at temperatures of 26°C/20°C (day/night) (a cycle of 26°C during daytime (15 hours) and 20°C during nighttime (9 hours)) under an artifically controlled environment. The rice plants were grown to a developing stage at 16 to 10 days before the heading timing and then subjected to low temperature treatment for 4 days at a temperature of 12°C (day and night). The treated rice plants were subsequently grown under the above environment at temperatures of 26°C/20°C (day/night) again. They underwent heading and flowering and then formed seeds. In this experiment, the transgenic rice plants were seeded in circular pots useful for seeding of 20 grains, and grown. A total of 100 mature individual rice plants per line were recorded for the day at which the panicles of each of them underwent heading. At a later date, how many days had passed until the day on which the relevant panicles underwent heading after completion of low temperature treatment (days taken until heading took place after low temperature treatment) was calculated. The seed fertility rate for each panicle was then calculated according to the above-described calculating formula, and then the average value of the rate of each line was calculated. The results are summarized as a graph in Fig. 6.

Average seed fertility rate per line was calculated via arithmetic averaging based on the seed fertility rate of each panicle that exhibited 10 to 16 days as the days taken until heading took place after low temperature treatment. The average seed fertility rates of the lines were 61.0% for the 122-1-1 line, 39.8% for the S33-5-2 line, and 43.0% for the S50-5-2 line. The average seed fertility rate of the original cultivar *"Yukihikari"* plants was 32.5%.

Therefore, it was demonstrated that transgenic rice plants into which the 1-SST gene or the 6-SFT gene had been introduced had high tolerance to cool temperatures to which the plants were exposed during their booting stage.

As described in detail above, in the case of rice plants, exposure to cool temperatures during their booting stage has the most significant influence in decreasing seed fertility. In the Examples, to examine the level of the influence of such exposure to cool temperatures during the most high-risk stage that has the most significant influence on the seed fertility, cool temperature treatment was provided to the rice plants at a stage 16 days to 10 days before heading (corresponding to the rice booting stage). As shown in Fig. 6, the transgenic rice plants of the present invention, into which the 1-SST gene or the 6-SFT gene had been introduced, showed higher seed fertility compared with those of the original cultivar plants, even when they had been undergone to cool temperature treatment during their booting stages. That is, it was demonstrated that the transgenic rice plants of the present invention can be cultivated at cool temperatures even during the most high-risk booting stage. Furthermore, it was considered that such average seed fertility calculated based on cool temperature treatment during the booting stage can be used as an appropriate indicator for testing the cool temperature resistance of the transgenic rice plants of the present-invention.

Industrial applicability

Transgenic rice plants according to the present invention, into which a fructosyltransferase gene has been introduced, can be used to make possible a rice cultivation by which chilling damage is alleviated in a region in which rice plants are often exposed to cool temperatures during the cultivation period. Furthermore, the method of the present invention for enhancing the chilling tolerance of rice plants can be employed to confer high-level chilling tolerance to rice plants in order to enable cultivation of existing rice plants with high yields under cool temperature conditions.

All publications, patents, and patent applications cited herein are incorporated herein by reference in their entirety.

Sequence listing free text

The sequences of SEQ ID NOS: 5 to 21 represent primers.

**Claims**

1. A transgenic rice plant having enhanced chilling tolerance, comprising at least one of the following fructosyltransferase genes (a) to (f):

   (a) a gene comprising the nucleotide sequence shown in SEQ ID NO: 1 or 3;
   (b) a gene comprising DNA that hybridizes under stringent conditions to DNA comprising the nucleotide sequence shown in SEQ ID NO: 1 or 3 and encodes a protein having fructan synthetic activity;
   (c) a gene encoding a protein that comprises the amino acid sequence shown in SEQ ID NO: 2 or 4;
   (d) a gene encoding a protein that comprises an amino acid sequence derived from the amino acid sequence shown in SEQ ID NO: 2 or 4 by deletion, substitution, or addition of one or several amino acids and has fructan synthetic activity;
   (e) a gene comprising a nucleotide sequence that exhibits 85% or more identity with the nucleotide sequence shown in SEQ ID NO: 1 or 3 and encoding a protein that has fructan synthetic activity; and
   (f) a gene encoding a protein that comprises an amino acid sequence exhibiting 85% or more identity with the amino acid sequence shown in SEQ ID NO: 2 or 4 and has fructan synthetic activity.

2. The transgenic rice plant according to claim 1, wherein the chilling tolerance is seedling-stage chilling tolerance and/or booting-stage chilling tolerance.

3. A method for enhancing chilling tolerance of a rice plant, comprising introducing at least one of the following fructosyltransferase genes (a) to (f) into a rice cell:

   (a) a gene comprising the nucleotide sequence shown in SEQ ID NO: 1 or 3;
   (b) a gene comprising DNA that hybridizes under stringent conditions to DNA comprising the nucleotide sequence shown in SEQ ID NO: 1 or 3 and encodes a protein having fructan synthetic activity;
   (c) a gene encoding a protein that comprises the amino acid sequence shown in SEQ ID NO: 2 or 4;
   (d) a gene encoding a protein that comprises an amino acid sequence derived from the amino acid sequence shown in SEQ ID NO: 2 or 4 by deletion, substitution, or addition of one or several amino acids and has fructan synthetic activity;
   (e) a gene comprising a nucleotide sequence that exhibits 85% or more identity with the nucleotide sequence shown in SEQ ID NO: 1 or 3 and encoding a protein that has fructan synthetic activity; and
   (f) a gene encoding a protein that comprises an amino acid sequence exhibiting 85% or more identity with the amino acid sequence shown in SEQ ID NO: 2 or 4 and has fructan synthetic activity.

**4.** The method according to claim 3, wherein the chilling tolerance is seedling-stage chilling tolerance and/or booting-stage chilling tolerance.

**5.** Use of the transgenic rice plant according to claim 1 or 2 for alleviating chilling damages in rice plant cultivation.

**6.** Use according to claim 5, wherein the chilling damages occur during seedling stage and/or booting stage of rice plants.

Fig. 1

A

Jerusalem artichoke tuber

B

Transgenic *Yukihikari* containing introduced 1-SST gene

C

*Yukihikari* (Control)

Time (min)

D

S :
1 :
2 :
3 :
4 :
5 :

: 2,1-linked fructose

EP 1 914 308 A1

Fig. 2

A — Transgenic *Yukihikari* containing introduced 1-SST gene

B — Transgenic *Yukihikari* containing introduced 6-SFT gene

C — *Yukihikari* (Control)

# Fig. 3

A

Stems of
winter wheat
(10 days after
flowering)

1K 6K

B

1

2

3

5

6

7

B

Leaves of
Transgenic *Yukihikari*
containing introduced
6-SFT gene

0        5        10       15       20       25       30       35

Time (min)

C

1K          6K

B           1

2           3

4           5

6           7

: 2,6-linked fructose

: 2,1-linked fructose

EP 1 914 308 A1

Fig. 4

I16   I22   I24   I29   I31   cont   S33   S50   S51

# Fig. 5

# Fig. 6

<div align="center">

**INTERNATIONAL SEARCH REPORT**

</div>

| International application No. |
|---|
| PCT/JP2006/312845 |

A. CLASSIFICATION OF SUBJECT MATTER
*C12N15/09*(2006.01)i, *A01H1/00*(2006.01)i, *A01H5/00*(2006.01)i

According to International Patent Classification (IPC) or to both national classification and IPC

B. FIELDS SEARCHED

Minimum documentation searched (classification system followed by classification symbols)
C12N15/09, A01H1/00, A01H5/00

Documentation searched other than minimum documentation to the extent that such documents are included in the fields searched

Electronic data base consulted during the international search (name of data base and, where practicable, search terms used)
WPI(STN), BIOSIS(STN), Medline(STN), AGRICOLA(STN), CROPU(STN), JSTPlus(JDreamII), GenBank/EMBL/DDBJ/GeneSeq,

C. DOCUMENTS CONSIDERED TO BE RELEVANT

| Category* | Citation of document, with indication, where appropriate, of the relevant passages | Relevant to claim No. |
|---|---|---|
| X | Akira KAWAKAMI, Midori YOSHIDA, "Wheat Fructan Gosei Koso Idenshi o Donyu shita Rice Keishitsu Tenkantai ni Chikuseki sareru Fructan no Kozo Kaiseki", Nippon Nogei Kagakukai 2005 Nendo Taikai Taikai Koen Yoshishu, 05 March, 2005 (05.03.05), page 35, (Abstract 29D070 β) | 1-6 |
| Y | HISANO, H. et al., Transgenic prennial ryegrass plants expressing wheat fructosyltransferase genes accumulate increased amounts of fructan and acquire increased tolerance on a cellular level to freezing., Plant Sci., 2004, Vol.167, pages 861 to 868 | 1-6 |

| ☒ | Further documents are listed in the continuation of Box C. | ☐ | See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 19 July, 2006 (19.07.06) | 25 July, 2006 (25.07.06) |

| Name and mailing address of the ISA/ | Authorized officer |
|---|---|
| Japanese Patent Office | |
| Facsimile No. | Telephone No. |

Form PCT/ISA/210 (second sheet) (April 2005)

**INTERNATIONAL SEARCH REPORT**

| International application No. |
| --- |
| PCT/JP2006/312845 |

C (Continuation).  DOCUMENTS CONSIDERED TO BE RELEVANT

| Category* | Citation of document, with indication, where appropriate, of the relevant passages | Relevant to claim No. |
| --- | --- | --- |
| Y | KAWAKAMI, A. and YOSHIDA, M., Molecular characterization of sucrose:sucrose 1-fructosyltransferase and sucrose:fructan 6-fructosyltransferase associated with fructan accumulation in winter wheat during cold hardening., Biosci.Biotechnol.Biochem., 2002, Vol.66, pages 2297 to 2305 | 1-6 |
| Y | KONSTANTINOVA T. et al., Freezing tolerant tobacco, transformed to accumulate osmoprotectants., Plant Sci., 2002, Vol.163, pages 157 to 164 | 1-6 |
| A | VINOCUR B. and ALTMAN A., Recent advances in engineering plant tolerance to abiotic stress: achievments in limitations., Curr.Opin. Biotechnol., 2005.4, Vol.16, pages 123 to 132 | 1-6 |
| A | US 6780623 B1 (KAWAKAMI, A. et al.), 24 August, 2004 (24.08.04), & JP 2000-350583 A | 1-6 |
| A | US 2002/0170086 A1 (ALLEN S.M. et al.), 14 November, 2002 (14.11.02), & US 6791015 B2       & US 2005/0010972 A1 | 1-6 |

Form PCT/ISA/210 (continuation of second sheet) (April 2005)

**INTERNATIONAL SEARCH REPORT**

International application No.

PCT/JP2006/312845

Continuation of B. FIELDS SEARCHED
  Electronic data base consulted during the international search (name of
data base and, where practicable, search terms used)

UniProt/SwissProt/PIR/PDB/GeneSeq

Form PCT/ISA/210 (extra sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000350583 A

- JP 2005182251 A


**Non-patent literature cited in the description**

- **KONSTANTINOVA T. et al.** *Plant Sci.,* 2002, vol. 163, 157-164
- **HISANO H. et al.** *Plant Sci.,* 2004, vol. 167, 861-868
- **KAWAKAMI A. ; YOSHIDA M.** *Biosci. Biotechnol. Biochem.,* 2002, vol. 66 (11), 2297-2305
- **TAJIMA et al.** Physiological Study on Inhibition of the Growth of Rice Due to Low Temperatures. *Agricultural Research Report (Nogikenho),* 1983, vol. 34, 69-111
- **SATAKE T. ; HAYASE H.** *Proc. Crop Sci. Japan,* 1970, vol. 39, 468-473
- **SAMBROOK et al.** Molecular Cloning, A Laboratory Manual. Cold Spring Harbor Laboratory Press, 2001
- **HIEI Y. et al.** fficient transformation of rice (Oryza sativa L.) mediated by Agrobacterium and sequence analysis of the boundaries of the T-DNA. *Plant J.,* 1994, vol. 6, 271-282

- **HAYASHIMOTO, A. et al.** A polyethylene glycol-mediated protoplast transformation system for production of fertile transgenic rice plants. *Plant Physiol.,* 1990, vol. 93, 857-863
- **CHRISTOU P et al.** *Biotechnology,* 1991, vol. 9, 957
- **GARHAM D. ; PATTERSON B. D.** *Ann. Rev. Plant Physiol.,* 1982, vol. 33, 347-372
- Science of Rice Plant Physiology. Physiology. vol. 2, 769-812
- **MITSUHARA 1. et al.** Efficient promoter cassettes for enhanced expression of foreign genes in dicotyledonous and monocotyledonous plant. *Plant Cell Physiol.,* 1996, vol. 37, 49-59
- **HIEI Y. ; OHTA S. ; KOMARI T. ; KUMASHIRO T.** Efficient transformation of rice (Oryza sativa L.) mediated by Agrobacterium and sequence analysis of the boundaries of the T-DNA. *Plant J.,* 1994, vol. 6, 271-282
- **SHIOMI, N.** *New Phytologist,* 1993, vol. 123, 263-270

SEQUENCE LISTING

<110> National Agriculture and Food Research Organization

<120> CHILLING TOLERANT PLANT AND METHOD FOR DEVELOPMENT OF THE SAME

<130> PH-2830-PCT

<140> PCT/JP2006/312845
<141> 2006-06-21

<150> JP 2005-182251
<151> 2005-06-22

<160> 21

<170> PatentIn Ver. 2.1

<210> 1
<211> 1989
<212> DNA
<213> Triticum aestivum L.

<220>
<223> Inventor: Yoshida, Midori; Kawakami, Akira; Sato, Yutaka

<220>
<221> CDS
<222> (1)..(1989)

<400> 1
```
atg gat tcg tct cgc gtc ata ctc atc ccc ggc acg ccg ccg ctg ccg    48
Met Asp Ser Ser Arg Val Ile Leu Ile Pro Gly Thr Pro Pro Leu Pro
 1               5                  10                  15

tac gcc tac gag cag ctg ccg tcc tcc tcc gcg gac gcc aag ggc atc    96
Tyr Ala Tyr Glu Gln Leu Pro Ser Ser Ser Ala Asp Ala Lys Gly Ile
                20                  25                  30

gag gag gag cgg gcc ggc ggc ggt ggc ctg agg tgg cgc gcg tgc gcc   144
Glu Glu Glu Arg Ala Gly Gly Gly Gly Leu Arg Trp Arg Ala Cys Ala
             35                  40                  45

gcc gtg ctg gcc gcc tcg gcc gtg gtg gcg ctc gtc gtc gcc gcc gcg   192
```

```
            Ala Val Leu Ala Ala Ser Ala Val Val Ala Leu Val Val Ala Ala Ala
                50                  55                  60

            gtc ttc ggg gcc agc ggg gcg ggc tgg gac gcg gtg gcc gcc tcc gtg    240
            Val Phe Gly Ala Ser Gly Ala Gly Trp Asp Ala Val Ala Ala Ser Val
                65                  70                  75                  80

            ccg gcg acc ccg gcg acg gag ttc ccg agg agc agg ggc aag gag cac    288
            Pro Ala Thr Pro Ala Thr Glu Phe Pro Arg Ser Arg Gly Lys Glu His
                            85                  90                  95

            ggc gtg tcg gag aag acg tcg ggg gcc tac tcc gcc aac gcg ttc ccg    336
            Gly Val Ser Glu Lys Thr Ser Gly Ala Tyr Ser Ala Asn Ala Phe Pro
                            100                 105                 110

            tgg agc aac gcc atg ctg cag tgg cag cgc acc ggc tac cat ttc cag    384
            Trp Ser Asn Ala Met Leu Gln Trp Gln Arg Thr Gly Tyr His Phe Gln
                    115                 120                 125

            ccg gac aag tac tac cag aac gat ccc aac ggt ccg gtt tac tat ggc    432
            Pro Asp Lys Tyr Tyr Gln Asn Asp Pro Asn Gly Pro Val Tyr Tyr Gly
                130                 135                 140

            gga tgg tac cac ttc ttc tac cag tac aac ccg tcg ggc tcc gtg tgg    480
            Gly Trp Tyr His Phe Phe Tyr Gln Tyr Asn Pro Ser Gly Ser Val Trp
            145                 150                 155                 160

            gag ccc caa atc gtg tgg ggc cac gcc gtg tcc aag gac ctc att cac    528
            Glu Pro Gln Ile Val Trp Gly His Ala Val Ser Lys Asp Leu Ile His
                            165                 170                 175

            tgg cgc cac ctc ccg ccg gcc ttg gtg ccc gac cag tgg tac gac atc    576
            Trp Arg His Leu Pro Pro Ala Leu Val Pro Asp Gln Trp Tyr Asp Ile
                        180                 185                 190

            aag ggc gtc ctc acc ggc tcc atc acc gtg ctc ccc gac ggc aag gtc    624
            Lys Gly Val Leu Thr Gly Ser Ile Thr Val Leu Pro Asp Gly Lys Val
                    195                 200                 205

            atc ctc ctc tac acg ggg aac acc gag acc ttt gcg cag gtg acc tgc    672
            Ile Leu Leu Tyr Thr Gly Asn Thr Glu Thr Phe Ala Gln Val Thr Cys
                210                 215                 220

            ctc gcg gag ccc gcc gac ccg agc gat ccc ctc ctc cgc gag tgg gtc    720
```

```
              Leu Ala Glu Pro Ala Asp Pro Ser Asp Pro Leu Leu Arg Glu Trp Val
              225                 230                 235                 240


aag cac ccc gcc aac ccc gtc gtg ttc ccg ccc ccc ggc atc ggc atg    768
Lys His Pro Ala Asn Pro Val Val Phe Pro Pro Pro Gly Ile Gly Met
                245                 250                 255


aag gac ttc cgc gac ccc acc acc gcg tgg ttc gac gag tcc gac ggc    816
Lys Asp Phe Arg Asp Pro Thr Thr Ala Trp Phe Asp Glu Ser Asp Gly
                260                 265                 270


acg tgg cgc acc atc atc ggc tcc aag aac gac tcg gac cac tcc ggc    864
Thr Trp Arg Thr Ile Ile Gly Ser Lys Asn Asp Ser Asp His Ser Gly
                275                 280                 285


atc gtc ttc tcc tac aag acc aag gac ttc ctc agc tac gag ctg atg    912
Ile Val Phe Ser Tyr Lys Thr Lys Asp Phe Leu Ser Tyr Glu Leu Met
                290                 295                 300


ccg ggg tac atg tac cgc ggc ccc aag ggc acc ggc gag tac gag tgc    960
Pro Gly Tyr Met Tyr Arg Gly Pro Lys Gly Thr Gly Glu Tyr Glu Cys
305                 310                 315                 320


atc gac ctc tac gcc gtc ggc ggg ggc cgc aag gcc agc gac atg tac   1008
Ile Asp Leu Tyr Ala Val Gly Gly Gly Arg Lys Ala Ser Asp Met Tyr
                325                 330                 335


aac tcg acg gcc gag gac gtg ctg tac gtg ctc aag gag agc agc gac   1056
Asn Ser Thr Ala Glu Asp Val Leu Tyr Val Leu Lys Glu Ser Ser Asp
                340                 345                 350


gac gac cgg cac gac tgg tac tcg ctg ggc cgg ttc gac gcc gcc gcc   1104
Asp Asp Arg His Asp Trp Tyr Ser Leu Gly Arg Phe Asp Ala Ala Ala
                355                 360                 365


aac aag tgg acg ccg atc gac gag gag ctg gag ctc ggc gtc ggg ctg   1152
Asn Lys Trp Thr Pro Ile Asp Glu Glu Leu Glu Leu Gly Val Gly Leu
                370                 375                 380


cgg tac gac tgg ggc aag tac tac gcg tcc aag tcc ttc tac gac ccc   1200
Arg Tyr Asp Trp Gly Lys Tyr Tyr Ala Ser Lys Ser Phe Tyr Asp Pro
385                 390                 395                 400


gtg aag aag cgg cgc gtc gtg tgg gcg tac gtc ggc gag acc gac tcg   1248
```

```
Val Lys Lys Arg Arg Val Val Trp Ala Tyr Val Gly Glu Thr Asp Ser
            405             410             415
```

```
gag cgc gcc gac atc acc aag ggg tgg gcc aac ctc cag tcg att ccg    1296
Glu Arg Ala Asp Ile Thr Lys Gly Trp Ala Asn Leu Gln Ser Ile Pro
            420             425             430
```

```
agg aca gtg gag ctt gac gag aag acc cgg acg aac ctc gtc caa tgg    1344
Arg Thr Val Glu Leu Asp Glu Lys Thr Arg Thr Asn Leu Val Gln Trp
            435             440             445
```

```
cct gtg gag gag ctc gat gcc ctc cgc atc aac acc acc gat ctc agc    1392
Pro Val Glu Glu Leu Asp Ala Leu Arg Ile Asn Thr Thr Asp Leu Ser
            450             455             460
```

```
ggc atc acc gtc ggc gcc ggc tcc gtt gcc ttc ctc ccc ctc cat cag    1440
Gly Ile Thr Val Gly Ala Gly Ser Val Ala Phe Leu Pro Leu His Gln
465             470             475             480
```

```
acc gct cag ctc gac atc gag gca acc ttc cgc atc gat gcc tcc gcc    1488
Thr Ala Gln Leu Asp Ile Glu Ala Thr Phe Arg Ile Asp Ala Ser Ala
            485             490             495
```

```
att gag gcc ctc aac gag gcc gat gtt agc tac aac tgc acc acc agc    1536
Ile Glu Ala Leu Asn Glu Ala Asp Val Ser Tyr Asn Cys Thr Thr Ser
            500             505             510
```

```
agc ggg gct gcc acc cgc ggc gcg ctt ggc ccc ttc ggc ctc ctt gtc    1584
Ser Gly Ala Ala Thr Arg Gly Ala Leu Gly Pro Phe Gly Leu Leu Val
            515             520             525
```

```
ctc gcc aac cgc gcc ctg acc gaa cag acg gga gtg tac ttc tat gtg    1632
Leu Ala Asn Arg Ala Leu Thr Glu Gln Thr Gly Val Tyr Phe Tyr Val
            530             535             540
```

```
tcc aag ggc ctc gac ggt ggt ctt cgg act cac ttc tgc cac gac gag    1680
Ser Lys Gly Leu Asp Gly Gly Leu Arg Thr His Phe Cys His Asp Glu
545             550             555             560
```

```
ttg cgc tcg tcg cat gct agt gac gtg gtg aag cgg gtg gtg ggt agc    1728
Leu Arg Ser Ser His Ala Ser Asp Val Val Lys Arg Val Val Gly Ser
            565             570             575
```

```
acg gtg cca gtg ctc gac ggc gaa gat ttt tcc gtt agg gtg ctc gtg    1776
```

```
            Thr Val Pro Val Leu Asp Gly Glu Asp Phe Ser Val Arg Val Leu Val
                        580             585             590


gac cac tcc att gtg cag agc ttc gcg atg ggc ggg agg ttg aca gca    1824
Asp His Ser Ile Val Gln Ser Phe Ala Met Gly Gly Arg Leu Thr Ala
            595             600             605


acg tcg agg gcg tac ccg acc gag gcc atc tac gcg gca gcg ggg gtc    1872
Thr Ser Arg Ala Tyr Pro Thr Glu Ala Ile Tyr Ala Ala Ala Gly Val
            610             615             620


tac atg ttc aac aac gcc acc ggc act agc gtc acc gcc gag aag ctt    1920
Tyr Met Phe Asn Asn Ala Thr Gly Thr Ser Val Thr Ala Glu Lys Leu
625             630             635             640


gtc gtg cat gat atg gac tcg tcg tac aac cat ata tac aca gat gat    1968
Val Val His Asp Met Asp Ser Ser Tyr Asn His Ile Tyr Thr Asp Asp
                645             650             655


gac ttg gta gtc gtc gat tag                                        1989
Asp Leu Val Val Val Asp
                660
```

```
<210> 2
<211> 662
<212> PRT
<213> Triticum aestivum L.

<400> 2
Met Asp Ser Ser Arg Val Ile Leu Ile Pro Gly Thr Pro Pro Leu Pro
1               5               10              15
Tyr Ala Tyr Glu Gln Leu Pro Ser Ser Ser Ala Asp Ala Lys Gly Ile
                20              25              30
Glu Glu Glu Arg Ala Gly Gly Gly Gly Leu Arg Trp Arg Ala Cys Ala
            35              40              45
Ala Val Leu Ala Ala Ser Ala Val Val Ala Leu Val Val Ala Ala Ala
        50              55              60
Val Phe Gly Ala Ser Gly Ala Gly Trp Asp Ala Val Ala Ala Ser Val
65              70              75              80
Pro Ala Thr Pro Ala Thr Glu Phe Pro Arg Ser Arg Gly Lys Glu His
                85              90              95
Gly Val Ser Glu Lys Thr Ser Gly Ala Tyr Ser Ala Asn Ala Phe Pro
                100             105             110
```

```
Trp Ser Asn Ala Met Leu Gln Trp Gln Arg Thr Gly Tyr His Phe Gln
        115                 120                 125
Pro Asp Lys Tyr Tyr Gln Asn Asp Pro Asn Gly Pro Val Tyr Tyr Gly
        130                 135                 140
Gly Trp Tyr His Phe Phe Tyr Gln Tyr Asn Pro Ser Gly Ser Val Trp
145                 150                 155                 160
Glu Pro Gln Ile Val Trp Gly His Ala Val Ser Lys Asp Leu Ile His
                165                 170                 175
Trp Arg His Leu Pro Pro Ala Leu Val Pro Asp Gln Trp Tyr Asp Ile
        180                 185                 190
Lys Gly Val Leu Thr Gly Ser Ile Thr Val Leu Pro Asp Gly Lys Val
        195                 200                 205
Ile Leu Leu Tyr Thr Gly Asn Thr Glu Thr Phe Ala Gln Val Thr Cys
210                 215                 220
Leu Ala Glu Pro Ala Asp Pro Ser Asp Pro Leu Leu Arg Glu Trp Val
225                 230                 235                 240
Lys His Pro Ala Asn Pro Val Val Phe Pro Pro Pro Gly Ile Gly Met
                245                 250                 255
Lys Asp Phe Arg Asp Pro Thr Thr Ala Trp Phe Asp Glu Ser Asp Gly
        260                 265                 270
Thr Trp Arg Thr Ile Ile Gly Ser Lys Asn Asp Ser Asp His Ser Gly
        275                 280                 285
Ile Val Phe Ser Tyr Lys Thr Lys Asp Phe Leu Ser Tyr Glu Leu Met
        290                 295                 300
Pro Gly Tyr Met Tyr Arg Gly Pro Lys Gly Thr Gly Glu Tyr Glu Cys
305                 310                 315                 320
Ile Asp Leu Tyr Ala Val Gly Gly Gly Arg Lys Ala Ser Asp Met Tyr
                325                 330                 335
Asn Ser Thr Ala Glu Asp Val Leu Tyr Val Leu Lys Glu Ser Ser Asp
        340                 345                 350
Asp Asp Arg His Asp Trp Tyr Ser Leu Gly Arg Phe Asp Ala Ala Ala
        355                 360                 365
Asn Lys Trp Thr Pro Ile Asp Glu Glu Leu Glu Leu Gly Val Gly Leu
        370                 375                 380
Arg Tyr Asp Trp Gly Lys Tyr Tyr Ala Ser Lys Ser Phe Tyr Asp Pro
385                 390                 395                 400
Val Lys Lys Arg Arg Val Val Trp Ala Tyr Val Gly Glu Thr Asp Ser
                405                 410                 415
Glu Arg Ala Asp Ile Thr Lys Gly Trp Ala Asn Leu Gln Ser Ile Pro
        420                 425                 430
Arg Thr Val Glu Leu Asp Glu Lys Thr Arg Thr Asn Leu Val Gln Trp
        435                 440                 445
Pro Val Glu Glu Leu Asp Ala Leu Arg Ile Asn Thr Thr Asp Leu Ser
450                 455                 460
```

22

```
Gly Ile Thr Val Gly Ala Gly Ser Val Ala Phe Leu Pro Leu His Gln
465             470             475             480
Thr Ala Gln Leu Asp Ile Glu Ala Thr Phe Arg Ile Asp Ala Ser Ala
                485             490             495
Ile Glu Ala Leu Asn Glu Ala Asp Val Ser Tyr Asn Cys Thr Thr Ser
            500             505             510
Ser Gly Ala Ala Thr Arg Gly Ala Leu Gly Pro Phe Gly Leu Leu Val
            515             520             525
Leu Ala Asn Arg Ala Leu Thr Glu Gln Thr Gly Val Tyr Phe Tyr Val
        530             535             540
Ser Lys Gly Leu Asp Gly Gly Leu Arg Thr His Phe Cys His Asp Glu
545             550             555             560
Leu Arg Ser Ser His Ala Ser Asp Val Val Lys Arg Val Val Gly Ser
            565             570             575
Thr Val Pro Val Leu Asp Gly Glu Asp Phe Ser Val Arg Val Leu Val
            580             585             590
Asp His Ser Ile Val Gln Ser Phe Ala Met Gly Gly Arg Leu Thr Ala
        595             600             605
Thr Ser Arg Ala Tyr Pro Thr Glu Ala Ile Tyr Ala Ala Ala Gly Val
        610             615             620
Tyr Met Phe Asn Asn Ala Thr Gly Thr Ser Val Thr Ala Glu Lys Leu
625             630             635             640
Val Val His Asp Met Asp Ser Ser Tyr Asn His Ile Tyr Thr Asp Asp
            645             650             655
Asp Leu Val Val Val Asp
        660
```

```
<210> 3
<211> 1851
<212> DNA
<213> Triticum aestivum L.

<220>
<221> CDS
<222> (1)..(1851)

<400> 3
atg ggg tca cac ggc aag cca ccg cta ccg tac gcg tac aag cca ctg      48
Met Gly Ser His Gly Lys Pro Pro Leu Pro Tyr Ala Tyr Lys Pro Leu
 1               5                  10                  15

ccc tcc gac gcc gac ggc gag cgg gcc ggc tgc acg agg tgg cgc gtg      96
```

```
Pro Ser Asp Ala Asp Gly Glu Arg Ala Gly Cys Thr Arg Trp Arg Val
        20                25                30

tgc gcc gtc gcg ctg acg gcc tcg gcc atg gtg gtg gtg gtg gtc ggc   144
Cys Ala Val Ala Leu Thr Ala Ser Ala Met Val Val Val Val Val Gly
        35                40                45

gcc acg ctc ctg gca ggg ttc cgg gtg gac cag gcc gtc gac gag gag   192
Ala Thr Leu Leu Ala Gly Phe Arg Val Asp Gln Ala Val Asp Glu Glu
    50                55                60

gcg gcg ggc ggg ttc ccg tgg agc aac gag atg ctg cag tgg cag cgc   240
Ala Ala Gly Gly Phe Pro Trp Ser Asn Glu Met Leu Gln Trp Gln Arg
65                70                75                80

agt ggc tac cat ttc cag acg gcc aag aac tac atg agc gat ccc aac   288
Ser Gly Tyr His Phe Gln Thr Ala Lys Asn Tyr Met Ser Asp Pro Asn
                85                90                95

ggt ctg atg tac tac cgt gga tgg tac cac atg ttc ttc cag tac aac   336
Gly Leu Met Tyr Tyr Arg Gly Trp Tyr His Met Phe Phe Gln Tyr Asn
            100               105               110

ccg gtg ggc acc gac tgg gac gac ggc atg gag tgg ggc cac gcc gtg   384
Pro Val Gly Thr Asp Trp Asp Asp Gly Met Glu Trp Gly His Ala Val
        115               120               125

tcc cgg aac ctc gtc caa tgg cgc acc ctc cct att gcc atg gtg gcc   432
Ser Arg Asn Leu Val Gln Trp Arg Thr Leu Pro Ile Ala Met Val Ala
        130               135               140

gac cag tgg tac gac atc ctc ggg gtt cta tcg ggc tct atg acc gtg   480
Asp Gln Trp Tyr Asp Ile Leu Gly Val Leu Ser Gly Ser Met Thr Val
145               150               155               160

cta ccc aat ggc acg gtc atc atg atc tac acg ggg gcc acc aac gcc   528
Leu Pro Asn Gly Thr Val Ile Met Ile Tyr Thr Gly Ala Thr Asn Ala
            165               170               175

tcc gcc gtt gag gtc cag tgc atc gcc act ccc gcc gac cct acc gac   576
Ser Ala Val Glu Val Gln Cys Ile Ala Thr Pro Ala Asp Pro Thr Asp
        180               185               190

ccc ctc ctc cgc cgc tgg acc aag cac ccc gcc aac ccc gtc atc tgg   624
```

Pro Leu Leu Arg Arg Trp Thr Lys His Pro Ala Asn Pro Val Ile Trp
      195             200             205

tcg ccg ccg ggg gtc ggc acc aag gat ttc cga gac ccg atg acc gct     672
Ser Pro Pro Gly Val Gly Thr Lys Asp Phe Arg Asp Pro Met Thr Ala
      210             215             220

tgg tac gat gaa tct gat gac aca tgg cgc acc ctg ctc ggg tcc aag     720
Trp Tyr Asp Glu Ser Asp Asp Thr Trp Arg Thr Leu Leu Gly Ser Lys
225             230             235             240

gac gac aac aac ggc cac cac gat ggc atc gcc atg atg tac aag acc     768
Asp Asp Asn Asn Gly His His Asp Gly Ile Ala Met Met Tyr Lys Thr
              245             250             255

aag gac ttc ctt aac tac gag ctc atc ccg ggc atc ttg cat cgg gtc     816
Lys Asp Phe Leu Asn Tyr Glu Leu Ile Pro Gly Ile Leu His Arg Val
          260             265             270

gag cgc acc ggc gag tgg gag tgc atc gac ttc tac cct gtc ggt cgc     864
Glu Arg Thr Gly Glu Trp Glu Cys Ile Asp Phe Tyr Pro Val Gly Arg
          275             280             285

cgc acc agc gac aac tca tcg gag atg ttg cac gtg ttg aag gcg agc     912
Arg Thr Ser Asp Asn Ser Ser Glu Met Leu His Val Leu Lys Ala Ser
      290             295             300

atg gac gac gaa cgg cat gac tac tac tcg cta ggc acg tac gac tct     960
Met Asp Asp Glu Arg His Asp Tyr Tyr Ser Leu Gly Thr Tyr Asp Ser
305             310             315             320

gcg gca aac agg tgg acg ccg atc gac ccg gag ctc gac ttg ggg atc     1008
Ala Ala Asn Arg Trp Thr Pro Ile Asp Pro Glu Leu Asp Leu Gly Ile
              325             330             335

ggg ttg aga tac gac tgg ggt aag ttc tac gcg tcc acc tcg ttc tat     1056
Gly Leu Arg Tyr Asp Trp Gly Lys Phe Tyr Ala Ser Thr Ser Phe Tyr
          340             345             350

gat ccg gcg aag aag cga cgc gtg ctg atg ggg tac gtc ggc gag gtc     1104
Asp Pro Ala Lys Lys Arg Arg Val Leu Met Gly Tyr Val Gly Glu Val
          355             360             365

gac tcc aag cgg gct gat gtg gtg aag gga tgg gcc tca att cag tca     1152

Asp Ser Lys Arg Ala Asp Val Val Lys Gly Trp Ala Ser Ile Gln Ser
    370             375             380

gtt cca agg aca att gct ctc gac gag aag acc cgg acg aac ctc ctc    1200
Val Pro Arg Thr Ile Ala Leu Asp Glu Lys Thr Arg Thr Asn Leu Leu
385             390             395             400

ctc tgg ccc gtg gag gag att gag acc ctc cgc ctc aac gcc acc gaa    1248
Leu Trp Pro Val Glu Glu Ile Glu Thr Leu Arg Leu Asn Ala Thr Glu
                405             410             415

ctc agc gac gtc acc ctt aac acc ggc tcc gtc atc cat atc ccg ctc    1296
Leu Ser Asp Val Thr Leu Asn Thr Gly Ser Val Ile His Ile Pro Leu
                420             425             430

cgc caa ggc act cag ctc gac atc gag gcc act ttc cac ctt gat gct    1344
Arg Gln Gly Thr Gln Leu Asp Ile Glu Ala Thr Phe His Leu Asp Ala
        435             440             445

tct gcc gtc gct gcc ctc aat gag gcc gat gtg ggc tac aac tgc agc    1392
Ser Ala Val Ala Ala Leu Asn Glu Ala Asp Val Gly Tyr Asn Cys Ser
    450             455             460

agc agc ggc ggt gct gtt aac cgc ggc gcg cta ggc ccc ttc ggc ctc    1440
Ser Ser Gly Gly Ala Val Asn Arg Gly Ala Leu Gly Pro Phe Gly Leu
465             470             475             480

ctc gtc ctc gct gcc ggt gac cgc cgt ggc gag caa acg gcg gtg tac    1488
Leu Val Leu Ala Ala Gly Asp Arg Arg Gly Glu Gln Thr Ala Val Tyr
                485             490             495

ttc tac gtg tcc agg ggg ctc gac gga ggc ctc cat acc agc ttc tgc    1536
Phe Tyr Val Ser Arg Gly Leu Asp Gly Gly Leu His Thr Ser Phe Cys
                500             505             510

caa gac gag tta cgg tcg tca cgg gcc aag gac gtg acg aag cga gtg    1584
Gln Asp Glu Leu Arg Ser Ser Arg Ala Lys Asp Val Thr Lys Arg Val
        515             520             525

att ggg agc acg gtg ccg gtg ctc gac ggc gag gct ttc tcg atg agg    1632
Ile Gly Ser Thr Val Pro Val Leu Asp Gly Glu Ala Phe Ser Met Arg
    530             535             540

gtg ctc gtg gac cac tcc atc gtg cag ggc ttc gcg atg ggc ggg agg    1680

```
Val Leu Val Asp His Ser Ile Val Gln Gly Phe Ala Met Gly Gly Arg
545                 550             555                 560

acc acg atg acg tca cgg gtg tac ccg atg gag gcc tat cag gag gca    1728
Thr Thr Met Thr Ser Arg Val Tyr Pro Met Glu Ala Tyr Gln Glu Ala
                565             570                 575

aaa gtg tac ttg ttc aac aat gcc acc ggt gcc agc gtc acg gcg gaa    1776
Lys Val Tyr Leu Phe Asn Asn Ala Thr Gly Ala Ser Val Thr Ala Glu
                580             585                 590

agg ctc gtc gtg cac gag atg gac tca gcg cac aac cag ctc tcc aat    1824
Arg Leu Val Val His Glu Met Asp Ser Ala His Asn Gln Leu Ser Asn
            595             600             605

atg gac gat cac tcg tat gtt caa tag                                1851
Met Asp Asp His Ser Tyr Val Gln
        610             615


<210> 4
<211> 616
<212> PRT
<213> Triticum aestivum L.


<400> 4
Met Gly Ser His Gly Lys Pro Pro Leu Pro Tyr Ala Tyr Lys Pro Leu
1               5               10              15
Pro Ser Asp Ala Asp Gly Glu Arg Ala Gly Cys Thr Arg Trp Arg Val
            20              25              30
Cys Ala Val Ala Leu Thr Ala Ser Ala Met Val Val Val Val Val Gly
            35              40              45
Ala Thr Leu Leu Ala Gly Phe Arg Val Asp Gln Ala Val Asp Glu Glu
        50              55              60
Ala Ala Gly Gly Phe Pro Trp Ser Asn Glu Met Leu Gln Trp Gln Arg
65              70              75                  80
Ser Gly Tyr His Phe Gln Thr Ala Lys Asn Tyr Met Ser Asp Pro Asn
                85              90              95
Gly Leu Met Tyr Tyr Arg Gly Trp Tyr His Met Phe Phe Gln Tyr Asn
            100             105             110
Pro Val Gly Thr Asp Trp Asp Asp Gly Met Glu Trp Gly His Ala Val
            115             120             125
Ser Arg Asn Leu Val Gln Trp Arg Thr Leu Pro Ile Ala Met Val Ala
        130             135             140
```

```
Asp Gln Trp Tyr Asp Ile Leu Gly Val Leu Ser Gly Ser Met Thr Val
145             150             155             160
Leu Pro Asn Gly Thr Val Ile Met Ile Tyr Thr Gly Ala Thr Asn Ala
            165             170             175
Ser Ala Val Glu Val Gln Cys Ile Ala Thr Pro Ala Asp Pro Thr Asp
            180             185             190
Pro Leu Leu Arg Arg Trp Thr Lys His Pro Ala Asn Pro Val Ile Trp
            195             200             205
Ser Pro Pro Gly Val Gly Thr Lys Asp Phe Arg Asp Pro Met Thr Ala
    210             215             220
Trp Tyr Asp Glu Ser Asp Asp Thr Trp Arg Thr Leu Leu Gly Ser Lys
225             230             235             240
Asp Asp Asn Asn Gly His His Asp Gly Ile Ala Met Met Tyr Lys Thr
            245             250             255
Lys Asp Phe Leu Asn Tyr Glu Leu Ile Pro Gly Ile Leu His Arg Val
            260             265             270
Glu Arg Thr Gly Glu Trp Glu Cys Ile Asp Phe Tyr Pro Val Gly Arg
    275             280             285
Arg Thr Ser Asp Asn Ser Ser Glu Met Leu His Val Leu Lys Ala Ser
    290             295             300
Met Asp Asp Glu Arg His Asp Tyr Tyr Ser Leu Gly Thr Tyr Asp Ser
305             310             315             320
Ala Ala Asn Arg Trp Thr Pro Ile Asp Pro Glu Leu Asp Leu Gly Ile
            325             330             335
Gly Leu Arg Tyr Asp Trp Gly Lys Phe Tyr Ala Ser Thr Ser Phe Tyr
            340             345             350
Asp Pro Ala Lys Lys Arg Arg Val Leu Met Gly Tyr Val Gly Glu Val
    355             360             365
Asp Ser Lys Arg Ala Asp Val Val Lys Gly Trp Ala Ser Ile Gln Ser
    370             375             380
Val Pro Arg Thr Ile Ala Leu Asp Glu Lys Thr Arg Thr Asn Leu Leu
385             390             395             400
Leu Trp Pro Val Glu Glu Ile Glu Thr Leu Arg Leu Asn Ala Thr Glu
            405             410             415
Leu Ser Asp Val Thr Leu Asn Thr Gly Ser Val Ile His Ile Pro Leu
            420             425             430
Arg Gln Gly Thr Gln Leu Asp Ile Glu Ala Thr Phe His Leu Asp Ala
    435             440             445
Ser Ala Val Ala Ala Leu Asn Glu Ala Asp Val Gly Tyr Asn Cys Ser
    450             455             460
Ser Ser Gly Gly Ala Val Asn Arg Gly Ala Leu Gly Pro Phe Gly Leu
465             470             475             480
Leu Val Leu Ala Ala Gly Asp Arg Arg Gly Glu Gln Thr Ala Val Tyr
            485             490             495
```

Phe Tyr Val Ser Arg Gly Leu Asp Gly Gly Leu His Thr Ser Phe Cys
500                     505                     510

Gln Asp Glu Leu Arg Ser Ser Arg Ala Lys Asp Val Thr Lys Arg Val
515                     520                     525

Ile Gly Ser Thr Val Pro Val Leu Asp Gly Glu Ala Phe Ser Met Arg
530                     535                     540

Val Leu Val Asp His Ser Ile Val Gln Gly Phe Ala Met Gly Gly Arg
545                     550                     555                     560

Thr Thr Met Thr Ser Arg Val Tyr Pro Met Glu Ala Tyr Gln Glu Ala
565                     570                     575

Lys Val Tyr Leu Phe Asn Asn Ala Thr Gly Ala Ser Val Thr Ala Glu
580                     585                     590

Arg Leu Val Val His Glu Met Asp Ser Ala His Asn Gln Leu Ser Asn
595                     600                     605

Met Asp Asp His Ser Tyr Val Gln
610                 615


<210> 5
<211> 18
<212> DNA
<213> Artificial Sequence

<220>
<223> Description of Artificial Sequence: primer

<400> 5
atggattcgt ctcgcgtc                                             18


<210> 6
<211> 21
<212> DNA
<213> Artificial Sequence

<220>
<223> Description of Artificial Sequence: primer

<400> 6
ctaatcgacg actaccaagt c                                         21


<210> 7

<211> 18
<212> DNA
<213> Artificial Sequence

<220>
<223> Description of Artificial Sequence: primer

<400> 7
atggggtcac acggcaag                                                    18


<210> 8
<211> 21
<212> DNA
<213> Artificial Sequence

<220>
<223> Description of Artificial Sequence: primer

<400> 8
tcattgaaca tacgagtgat c                                                21


<210> 9
<211> 17
<212> DNA
<213> Artificial Sequence

<220>
<223> Description of Artificial Sequence: primer

<220>
<221> modified base
<222> 12
<223> n represents a, c, g, or t

<400> 9
atgaaygayc cnaaygg                                                     17


<210> 10
<211> 17
<212> DNA
<213> Artificial Sequence

```
<220>
<223> Description of Artificial Sequence: primer


<220>
<221> modified base
<222> 3 and 6
<223> n represents a, c, g, or t


<400> 10
ccngtngcrt trttraa                                                    17



<210> 11
<211> 29
<212> DNA
<213> Artificial Sequence


<220>
<223> Description of Artificial Sequence: primer


<400> 11
ttggatccat tatggattcg tctcgcgtc                                       29



<210> 12
<211> 29
<212> DNA
<213> Artificial Sequence


<220>
<223> Description of Artificial Sequence: primer


<400> 12
aagagctcct aatcgacgac taccaagtc                                       29



<210> 13
<211> 29
<212> DNA
<213> Artificial Sequence


<220>
<223> Description of Artificial Sequence: primer
```

<400> 13
ctggatccaa gatggggtca cacggcaag                                    29


<210> 14
<211> 29
<212> DNA
<213> Artificial Sequence

<220>
<223> Description of Artificial Sequence: primer

<400> 14
ccgagctctc attgaacata cgagtgatc                                    29


<210> 15
<211> 28
<212> DNA
<213> Artificial Sequence

<220>
<223> Description of Artificial Sequence: primer

<400> 15
ggtaagtaat tgctactggt atcacttg                                     28


<210> 16
<211> 21
<212> DNA
<213> Artificial Sequence

<220>
<223> Description of Artificial Sequence: primer

<400> 16
ggagcccgac ggggtgtact g                                            21


<210> 17
<211> 18
<212> DNA

<213> Artificial Sequence

<220>
<223> Description of Artificial Sequence: primer

<400> 17
ctcaacggcg gaggcgtt                                                    18


<210> 18
<211> 21
<212> DNA
<213> Artificial Sequence

<220>
<223> Description of Artificial Sequence: primer

<400> 18
gccaacgcgt tcccgtggag c                                                21


<210> 19
<211> 29
<212> DNA
<213> Artificial Sequence

<220>
<223> Description of Artificial Sequence: primer

<400> 19
atcgacgact accaagtcat catctgtgt                                        29


<210> 20
<211> 23
<212> DNA
<213> Artificial Sequence

<220>
<223> Description of Artificial Sequence: primer

<400> 20
gcgggcgggt tcccgtggag caa                                              23

```
<210> 21
<211> 31
<212> DNA
<213> Artificial Sequence

<220>
<223> Description of Artificial Sequence: primer

<400> 21
ttgaacatac gagtgatcgt ccatattgga g                                    31
```